# EUROPEAN PATENT APPLICATION

(11) **EP 4 032 551 A1**
(43) Date of publication of application: **27.07.2022**
(21) Application number: 20864622.4
(22) Date of filing: 16.09.2020
(51) Int. Cl.: A61K 48/00, A61P 25/00, A61P 43/00, C12N 5/079, C12N 5/10, A61K 47/54, C12N 15/113, A61K 31/713

(54) **NUCLEIC ACID COMPLEX**

(30) Priority: 18.09.2019 JP 2019168930
(71) Applicant: National University Corporation Tokyo Medical and Dental University, Tokyo 113-8510 (JP); Takeda Pharmaceutical Company Limited, Osaka-shi, Osaka 541-0045 (JP)
(72) Inventor: YOKOTA, Takanori, Tokyo 113-8510 (JP); NAGATA, Tetsuya, Tokyo 113-8510 (JP); FURUKAWA, Hideki, Fujisawa-shi, Kanagawa 251-0012 (JP); YOGO, Takatoshi, Fujisawa-shi, Kanagawa 251-0012 (JP); NAKAGAWA, Yasuo, Fujisawa-shi, Kanagawa 251-0012 (JP); SASAKI, Shigekazu, Fujisawa-shi, Kanagawa 251-0012 (JP); TOKUNOH, Ryosuke, Fujisawa-shi, Kanagawa 251-0012 (JP); SEKI, Tomohiro, Fujisawa-shi, Kanagawa 251-0012 (JP); HIDAKA, Kosuke, Fujisawa-shi, Kanagawa 251-0012 (JP); KIKUCHI, Fumiaki, Fujisawa-shi, Kanagawa 251-0012 (JP); KUBO, Osamu, Fujisawa-shi, Kanagawa 251-0012 (JP); KASAHARA, Takahito, Fujisawa-shi, Kanagawa 251-0012 (JP); KOJIMA, Takuto, Fujisawa-shi, Kanagawa 251-0012 (JP); WANG, Junsi, Fujisawa-shi, Kanagawa 251-0012 (JP); TOKUNAGA, Norihito, Fujisawa-shi, Kanagawa 251-0012 (JP)
(74) Representative: Jones, Nicholas Andrew
(86) International application number: PCT/JP2020/035117
(87) International publication number: WO 2021/054370

(57) **Abstract**

The object of the present invention is to provide a nucleic acid agent that is efficiently delivered to the nervous system, for example, the central nervous system to which drug delivery can be prevented by BBB, and produces an antisense effect on the target transcriptional product at the delivery site, and a composition comprising the same. In an embodiment, the present invention provides a double-stranded nucleic acid complex formed by annealing a first nucleic acid strand that hybridizes to a part of a target transcriptional product and has an antisense effect on the target transcriptional product, and a second nucleic acid strand that comprises a base sequence complementary to the first nucleic acid strand and is bound to a C₂₂₋₃₅ alkyl group optionally substituted with a hydroxy group or an analog thereof.

SEQUENCE LISTING

<110>  National University Corporation Tokyo Medical and Dental
       University
       TAKEDA PHARMACEUTICAL COMPANY LIMITED

<120>  Nucleic acid complex

<130>  PH-8528-PCT

<150>  JP 2019-168930
<151>  2019-09-18

<160>  8

<170>  PatentIn version 3.5

<210>  1
<211>  16
<212>  DNA
<213>  Artificial

<220>
<223>  Synthetic

<220>
<221>  misc_feature
<222>  (2)..(3), (14)..(15)
<223>  LNA

<220>
<221>  misc_feature
<222>  (1)..(1), (16)..(16)
<223>  5-methylcytosine LNA

<400>  1
ctagttcact gaatgc                                                       16


<210>  2
<211>  16
<212>  RNA
<213>  Artificial

<220>
<223>  Synthetic

<220>
<221>  misc_feature
<222>  (1)..(3), (14)..(16)
<223>  2'-O-Me RNA

<400>  2
gcauucagug aacuag                                                       16


<210>  3
<211>  6982
<212>  DNA
<213>  Mus musculus

<220>
<223>  Malat1 non-coding RNA

<400> 3
aggcattcag gcagcgagag cagagcagcg tagagcagca cagctgagct cgtgaggcag 60
gagactcagc ccgaggaaat cgcagataag tttttaatta aaaagattga gcagtaaaaa 120
gaattagaac tctaaactta agctaataga gtagcttatc gaaatattac ttagtcttaa 180
taatctaaga agatcttaag agataacatg aaggcttatt taaacagttt gaaaaaggaa 240
atgaggagaa aagtatttgt actgtataat ggaggctgac cagagcagtt taggagattg 300
taaagggagg ttttgtgaag ttctaaaagg ttctagtttg aaggtcggcc ttgtagatta 360
aaacgaaggt tacctaaata gaatctaagt ggcatttaaa acagtaaagt tgtagagaat 420
agtttgaaaa tgaggtgtag ttttaaaaga ttgagaaaag taggttaagt tgacggccgt 480
tataaaaatc cttcgactgg cgcatgtacg tttgaaggca tgagttggaa acagggaaga 540
tggaagtgtt aggctagccg ggcgatggtg gcgcacgcct ttaatcctag cacttgggag 600
gcagaggcag gcggatttct gagttcgagg ccagcctggt ctacagagtg agttccagga 660
cagccagggc tacacagaga aaccctgtct tgaaaaaaca aaaaggttag gctagtattt 720
ggagaaagaa gattagaaaa tggaagtgaa agacgaagaa gacatacagg aaggtgaaga 780
aaaagctgtt agagaagata ggaaaataga agacaaagca tctttagaag acagaaaagg 840
tacttaaagg cacaggtagt aggaagccga agaatagaag atagaaagaa gcaagataga 900
aaaacaaaat ggaagttaag acaactttgg atgccagcat tcaagatagg caaagaagat 960
aagattgagg ccaaaaggtt ggataagata taaagtcaga aggaaattat ctttaaagcc 1020
ataagttcaa atttctgatg gagcgagcag tttagaagag tctttagaca gccacataca 1080
agattgaagc tagcaatcaa agctactagg actgaagtaa aaagttaagg cagaatgcct 1140
ttgaagagtt agaagaatat taaaagcctt aacttgtagc ttaattttgc ttgatgacaa 1200
aaggactttt gataacagtt tcaagattgt cagcattttg cattggactt gagctgaggt 1260
gcttttaaaa tcctaacgac tagcattggc agctgaccca ggtctacaca gaagtgcatt 1320
cagtgaacta ggaagacagg agcggcagac aggagtcccg aagccagttt ggtgaagcta 1380
ggaaggactg aggagccagc agcagcagtg catggtgaag atagcccagg aaagagtgcg 1440
gttcggtgga ggaagctagg aagaaggagc catacggatg tggtggtgaa gctgggaaag 1500
ggttccagga tggtggagcg agagcgagtt ggtgatgaag ctagctggcg gcttggcttg 1560
tcaactgcgc ggaggaggcg agcaggcatt gtggagagga tagatagcgg ctcctagacc 1620
agcatgccag tgtgcaagaa aggctgcagg gagagcatgc ggtgcggtaa cattccttga 1680
ggtcggcaac atggtggtgg ttttctgtaa cttggatggt aacttgttta ctttgtctta 1740
atagttatgg gggagttgta ggcttctgtg taaagagata tatctggggc tgtatgtagg 1800
cctttgcggg tgttgtaggt ttttcttttt cagggttatg tcctcttgca tcttgtcaga 1860

agcttttgag ggctgactgc caaggcccag aaagaagaat ggtagatggc aagttgtctt 1920

taaccgctca gaggggaatg aatggtagag ccagcacaac ctcccagttt tgtaagacgt 1980

tgtagtttga acagatgacc taccacaagc ctcactcctg tgtaggggag gtaattgggc 2040

aaagtgcttt tgggggaatg ggggcaaaat atattttgag ttcttttccc cttaggtctg 2100

tctagaatcc taaaggcaga tgactcaagg gaaccagaaa aaaggaaatc cactctcagg 2160

ataagcagag ctcgccaggt ttacagtttg taggaagtag aggatggatg ctagctttca 2220

cactgagtgt ggaggagctg gccatggcgg aattgctggt agtttactct ttccccctcc 2280

cttaatgaga tttgtaaaat cctaaacact tttacttgaa atatttggga gtggtcttaa 2340

cagggaggag tgggtggggg aaacgttttt tttctaagat tttccacaga tgctatagtt 2400

gtgttgacac actgggttag agaaggcgtg tactgctatg ctgttggcac gacaccttca 2460

gggactggag ctgccttttg tccttggaag agttttccca gttgccgctg aagtcagcac 2520

agtgcggctt tggttcacag tcacctcagg agaacctcag gagcttggct aggccagagg 2580

ttgaagttaa gttttacagc accgtgattt aaaatatttc attaaagggg aggggtaaaa 2640

cttagttggc tgtggccttg tgtttgggtg ggtgggggtg ttaggtaatt gtttagttta 2700

tgatttcaga taatcatacc agagaactta aatatttgga aaaacaggaa atctcagctt 2760

tcaagttggc aagtaactcc caatccagtt tttgcttctt ttttcctttt tctttttttg 2820

aggcgggcag ctaaggaagg ttggttcctc tgccggtccc tcgaaagcgt agggcttggg 2880

ggttggtctg gtccactggg atgatgtgat gctacagtgg ggactcttct gaagctgttg 2940

gatgaatata gattgtagtg tgtggttctc ttttgaaatt tttttcaggt gacttaatgt 3000

atcttaataa ctactatagg aacaaaggaa gtggctttaa tgaccctgaa ggaatttctt 3060

ctggtgatag cttttatatt atcaagtaag agatactatc tcagttttgt ataagcaagt 3120

ctttttccta gtgtaggaga aatgatttcc cttgtgacta aacaagatgt aaaggtatgc 3180

tttttttctt cttgtgcatt gtatacttgt gtttatttgt aacttataat ttaagaatta 3240

tgataattca gcctgaatgt cttttagagg gtgggctttt gttgatgagg gaggggaaac 3300

cttttttttt ctgtagacct ttttcagata acaccatctg agtcataacc agcctggcag 3360

tgtgatgacg tagatgcaga gggagcagct ccttggtgaa tgagtgataa gtaaaggcag 3420

aaaaaataat gtcatgtctc catggggaat gagcatgagc cagagattgt tcctactgat 3480

gaaaagctgc atatgcaaaa atttaagcaa atgaaagcaa ccagtataaa gttatggcaa 3540

tacctttaaa agttatggct tatctaccaa gctttatcca caaaagtaaa gaattgatga 3600

aaaacagtga agatcaaatg ttcatctcaa aactgctttt acaaaagcag aatagaaatg 3660

aagtgaaaat gctgcattaa gcctggagta aaaagaagct gagcttgttg agatgagtgg 3720

gatcgagcgg ctgcgaggcg gtgcagtgtg ccaatgtttc gtttgcctca gacaggtttc 3780

tcttcataag cagaagagtt gcttcattcc atctcggagc aggaaacagc agactgctgt 3840

tgacagataa gtgtaacttg gatctgcagt attgcatgtt agggatagat aagtgccttt 3900

tttctctttt tccaaaaaga cctgtagagc tgttgaatgt ttgcagctgg cccctcttag 3960

gcagttcaga attttgagta gttttcccat ccagcctctt aaaaattcct aagccttgca 4020

ccgatgggct ttcatgatgg gatagctaat aggcttttgc atcgtaaact tcaacacaaa 4080

agcctacatg attaatgcct actttaatta cattgcttac aagattaagg aatctttatc 4140

ttgaagaccc catgaaaggg atcattatgt gctgaaaatt agatgttcat attgctaaaa 4200

tttaaatgtg ctccaatgta cttgtgctta aaatcattaa attatacaaa ttaataaaat 4260

acttcactag agaatgtatg tatttagaag gctgtctcct tatttaaata aagtcttgtt 4320

tgttgtctgt agttagtgtg ggcaattttg gggggatgtt cttctctaat cttttcagaa 4380

acttgacttc gaacacttaa gtggaccaga tcaggatttg agccagaaga ccgaaattaa 4440

ctttaaggca ggaaagacaa attttattct ccatgcagtg atgagcattt aataattgca 4500

ggcctggcat agaggccgtc taactaagga ctaagtacct taggcaggtg ggagatgatg 4560

gtcagagtaa aaggtaacta catattttgt ttccagaaag tcaggggtct aatttgacca 4620

tggctaaaca tctagggtaa gacacttttc ccccacattt ccaaatatgc atgttgagtt 4680

taaatgctta cgatcatctc atccacttta gccttttgtc acctcacttg agccacgagt 4740

ggggtcaggc atgtgggttt aaagagtttt cctttgcaga gcctcatttc atccttcatg 4800

gagctgctca ggactttgca tataagcgct tgcctctgtc ttctgttctg ctagtgagtg 4860

tgtgatgtga gaccttgcag tgagtttgtt tttcctggaa tgtggaggga gggggggatg 4920

gggcttactt gttctagctt tttttttaca gaccacacag aatgcaggtg tcttgacttc 4980

aggtcatgtc tgttctttgg caagtaatat gtgcagtact gttccaatct gctgctatta 5040

gaatgcattg tgacgcgact ggagtatgat taaagaaagt tgtgtttccc caagtgtttg 5100

gagtagtggt tgttggagga aaagccatga gtaacaggct gagtgttgag gaaatggctc 5160

tctgcagctt taagtaaccc gtgtttgtga ttggagccga gtccctttgc tgtgctgcct 5220

taggtaaatg tttttgttca tttctggtga ggggggttgg gagcactgaa gcctttagtc 5280

tcttccagat tcaacttaaa atctgacaag aaataaatca gacaagcaac attcttgaag 5340

aaattttaac tggcaagtgg aaatgttttg aacagttccg tggtctttag tgcattatct 5400

ttgtgtaggt gttctctctc ccctcccttg gtcttaattc ttacatgcag gaacattgac 5460

aacagcagac atctatctat tcaaggggcc agagaatcca gacccagtaa ggaaaaatag 5520

cccatttact ttaaatcgat aagtgaagca gacatgccat tttcagtgtg gggattggga 5580

agccctagtt ctttcagatg tacttcagac tgtagaagga gcttccagtt gaattgaaat 5640

tcaccagtgg acaaaatgag gacaacaggt gaacgagcct tttcttgttt aagattagct 5700

actggtaatc tagtgttgaa tcctctccag cttcatgctg gagcagctag catgtgatgt 5760

aatgttggcc ttggggtgga ggggtgaggt gggcgctaag ccttttttta agatttttca 5820

ggtacccctc actaaaggca ctgaaggctt aatgtaggac agcggagcct tcctgtgtgg 5880

caagaatcaa gcaagcagta ttgtatcgag accaaagtgg tatcatggtc ggttttgatt 5940

agcagtgggg actaccctac cgtaacacct tgttggaatt gaagcatcca aagaaaatac 6000

ttgagaggcc ctgggcttgt tttaacatct ggaaaaaagg ctgtttttat agcagcggtt 6060

accagcccaa acctcaagtt gtgcttgcag gggagggaaa agggggaaag cgggcaacca 6120

gtttccccag cttttccaga atcctgttac aaggtctccc cacaagtgat ttctctgcca 6180

catcgccacc atgggccttt ggcctaatca cagacccttc acccctcacc ttgatgcagc 6240

cagtagctgg atccttgagg tcacgttgca tatcggtttc aaggtaacca tggtgccaag 6300

gtcctgtggg ttgcaccaga aaaggccatc aattttcccc ttgcctgtaa tttaacatta 6360

aaaccatagc taagatgttt tatacatagc acctatgcag agtaaacaaa ccagtatggg 6420

tatagtatgt ttgataccag tgctgggtgg gaatgtagga agtcggatga aaagcaagcc 6480

tttgtaggaa gttgttgggg tgggattgca aaaattctct gctaagactt tttcaggtgg 6540

acataacaga cttggccaag ctagcatctt agtggaagca gattcgtcag tagggttgta 6600

aaggtttttc ttttcctgag aaaacaacct tttgttttct caggttttgc tttttggcct 6660

ttccctagct ttaaaaaaaa aaaagcaaaa gacgctggtg gctggcactc ctggtttcca 6720

ggacggggtt caagtccctg cggtgtcttt gcttgactct tatatcatga ggccattaca 6780

tttttcttgg agggttctaa aggctctggg tatggtagct gatatcactg gaacactccc 6840

cagcctcagt gttgaactct tgataattaa ctgcattgtc tttcaggtta tgcccaattc 6900

gtcttattac ctctgagtcg acacacctcc tactatttat tgaatacttt gattttatga 6960

aataaaaact aaatatctct ca 6982

<210> 4
<211> 8758
<212> DNA
<213> Homo sapiens

<220>
<223> Malat1 non-coding RNA

<400> 4
   gtaaaggact ggggccccgc aactggcctc tcctgccctc ttaagcgcag cgccatttta 60
   gcaacgcaga agcccggcgc cgggaagcct cagctcgcct gaaggcaggt cccctctgac 120
   gcctccggga gcccaggttt cccagagtcc ttgggacgca gcgacgagtt gtgctgctat 180
   cttagctgtc cttataggct ggccattcca ggtggtggta tttagataaa accactcaaa 240
   ctctgcagtt tggtcttggg gtttggagga aagcttttat ttttcttcct gctccggttc 300
   agaaggtctg aagctcatac ctaaccaggc ataacacaga atctgcaaaa caaaaacccc 360
   taaaaaagca gacccagagc agtgtaaaca cttctgggtg tgtccctgac tggctgccca 420
   aggtctctgt gtcttcggag acaaagccat tcgcttagtt ggtctacttt aaaaggccac 480
   ttgaactcgc tttccatggc gatttgcctt gtgagcactt tcaggagagc ctggaagctg 540
   aaaaacggta gaaaaatttc cgtgcggccc gtggggggct ggcggcaact ggggggccgc 600
   agatcagagt gggccactgg cagccaacgg cccccggggc tcaggcgggg agcagctctg 660
   tggtgtggga ttgaggcgtt ttccaagagt gggttttcac gtttctaaga tttcccaagc 720
   agacagcccg tgctgctccg atttctcgaa caaaaaagca aaacgtgtgg ctgtcttggg 780
   agcaagtcgc aggactgcaa gcagttgggg gagaaagtcc gccattttgc cacttctcaa 840
   ccgtccctgc aaggctgggg ctcagttgcg taatggaaag taaagccctg aactatcaca 900
   ctttaatctt ccttcaaaag gtggtaaact atacctactg tccctcaaga gaacacaaga 960
   agtgctttaa gaggtatttt aaaagttccg ggggttttgt gaggtgtttg atgacccgtt 1020
   taaaatatga tttccatgtt tcttttgtct aaagtttgca gctcaaatct ttccacacgc 1080
   tagtaattta agtatttctg catgtgtagt ttgcattcaa gttccataag ctgttaagaa 1140
   aaatctagaa aagtaaaact agaacctatt tttaaccgaa gaactacttt ttgcctccct 1200
   cacaaaggcg gcggaaggtg atcgaattcc ggtgatgcga gttgttctcc gtctataaat 1260
   acgcctcgcc cgagctgtgc ggtaggcatt gaggcagcca gcgcaggggc ttctgctgag 1320
   ggggcaggcg gagcttgagg aaaccgcaga taagtttttt tctctttgaa agatagagat 1380
   taatacaact acttaaaaaa tatagtcaat aggttactaa gatattgctt agcgttaagt 1440
   ttttaacgta attttaatag cttaagattt taagagaaaa tatgaagact tagaagagta 1500
   gcatgaggaa ggaaaagata aaaggtttct aaaacatgac ggaggttgag atgaagcttc 1560
   ttcatggagt aaaaaatgta tttaaaagaa aattgagaga aaggactaca gagccccgaa 1620
   ttaataccaa tagaagggca atgcttttag attaaaatga aggtgactta aacagcttaa 1680
   agtttagttt aaaagttgta ggtgattaaa ataatttgaa ggcgatcttt taaaaagaga 1740
   ttaaaccgaa ggtgattaaa agaccttgaa atccatgacg cagggagaat tgcgtcattt 1800
   aaagcctagt taacgcattt actaaacgca gacgaaaatg gaaagattaa ttgggagtgg 1860
   taggatgaaa caatttggag aagatagaag tttgaagtgg aaaactggaa gacagaagta 1920
   cgggaaggcg aagaaaagaa tagagaagat agggaaatta gaagataaaa acatactttt 1980
   agaagaaaaa agataaattt aaacctgaaa agtaggaagc agaagaaaaa agacaagcta 2040
   ggaaacaaaa agctaagggc aaaatgtaca aacttagaag aaaattggaa gatagaaaca 2100
   agatagaaaa tgaaaatatt gtcaagagtt tcagatagaa aatgaaaaac aagctaagac 2160
   aagtattgga gaagtataga agatagaaaa atataaagcc aaaaattgga taaaatagca 2220
   ctgaaaaaat gaggaaatta ttggtaacca atttatttta aaagcccatc aatttaattt 2280
   ctggtggtgc agaagttaga aggtaaagct tgagaagatg agggtgttta cgtagaccag 2340
   aaccaattta gaagaatact tgaagctaga aggggaagtt ggttaaaaat cacatcaaaa 2400
   agctactaaa aggactggtg taatttaaaa aaaactaagg cagaaggctt ttggaagagt 2460
   tagaagaatt tggaaggcct taaatatagt agcttagttt gaaaaatgtg aaggactttc 2520
   gtaacggaag taattcaaga tcaagagtaa ttaccaactt aatgtttttg cattggactt 2580
   tgagttaaga ttatttttta aatcctgagg actagcatta attgacagct gacccaggtg 2640
   ctacacagaa gtggattcag tgaatctagg aagacagcag cagacaggat tccaggaacc 2700
   agtgtttgat gaagctagga ctgaggagca agcgagcaag cagcagttcg tggtgaagat 2760
   aggaaaagag tccaggagcc agtgcgattt ggtgaaggaa gctaggaaga aggaaggagc 2820
   gctaacgatt tggtggtgaa gctaggaaaa aggattccag gaaggagcga gtgcaatttg 2880
   gtgatgaagg tagcaggcgg cttggcttgg caaccacacg gaggaggcga gcaggcgttg 2940
   tgcgtagagg atcctagacc agcatgccag tgtgccaagg ccacagggaa agcgagtggt 3000
   tggtaaaaat ccgtgaggtc ggcaatatgt tgtttttctg gaacttactt atggtaacct 3060
   tttatttatt ttctaatata atgggggagt ttcgtactga ggtgtaaagg gatttatatg 3120
   gggacgtagg ccgatttccg ggtgttgtag gtttctcttt ttcaggctta tactcatgaa 3180
   tcttgtctga agcttttgag ggcagactgc caagtcctgg agaaatagta gatggcaagt 3240
   ttgtgggttt ttttttttta cacgaatttg aggaaaacca aatgaatttg atagccaaat 3300
   tgagacaatt tcagcaaatc tgtaagcagt ttgtatgttt agttggggta atgaagtatt 3360
   tcagttttgt gaatagatga cctgttttta cttcctcacc ctgaattcgt tttgtaaatg 3420
   tagagtttgg atgtgtaact gaggcggggg ggagttttca gtattttttt ttgtgggggt 3480
   gggggcaaaa tatgttttca gttctttttc ccttaggtct gtctagaatc ctaaaggcaa 3540
   atgactcaag gtgtaacaga aaacaagaaa atccaatatc aggataatca gaccaccaca 3600
   ggtttacagt ttatagaaac tagagcagtt ctcacgttga ggtctgtgga agagatgtcc 3660
   attggagaaa tggctggtag ttactctttt ttccccccac ccccttaatc agactttaaa 3720
   agtgcttaac cccttaaact tgttattttt tacttgaagc attttgggat ggtcttaaca 3780
   gggaagagag agggtggggg agaaaatgtt tttttctaag attttccaca gatgctatag 3840
   tactattgac aaactgggtt agagaaggag tgtaccgctg tgctgttggc acgaacacct 3900
   tcagggactg gagctgcttt tatccttgga agagtattcc cagttgaagc tgaaaagtac 3960
   agcacagtgc agctttggtt catattcagt catctcagga gaacttcaga agagcttgag 4020
   taggccaaat gttgaagtta agttttccaa taatgtgact tcttaaaagt tttattaaag 4080
   gggaggggca aatattggca attagttggc agtggcctgt tacggttggg attggtgggg 4140
   tgggtttagg taattgttta gtttatgatt gcagataaac tcatgccaga gaacttaaag 4200
   tcttagaatg gaaaaagtaa agaaatatca acttccaagt tggcaagtaa ctcccaatga 4260
   tttagttttt ttccccccag tttgaattgg gaagctgggg gaagttaaat atgagccact 4320
   gggtgtacca gtgcattaat ttgggcaagg aaagtgtcat aatttgatac tgtatctgtt 4380
   ttccttcaaa gtatagagct tttggggaag gaaagtattg aactgggggt tggtctggcc 4440
   tactgggctg acattaacta caattatggg aaatgcaaaa gttgtttgga tatggtagtg 4500
   tgtggttctc ttttggaatt tttttcaggt gatttaataa taatttaaaa ctactataga 4560
   aactgcagag caaaggaagt ggcttaatga tcctgaaggg atttcttctg atggtagctt 4620
   ttgtattatc aagtaagatt ctattttcag ttgtgtgtaa gcaagttttt ttttagtgta 4680
   ggagaaatac ttttccattg tttaactgca aaacaagatg ttaaggtatg cttcaaaaat 4740
   tttgtaaatt gtttatttta aacttatctg tttgtaaatt gtaactgatt aagaattgtg 4800
   atagttcagc ttgaatgtct cttagagggt gggcttttgt tgatgaggga ggggaaactt 4860
   tttttttttc tatagacttt tttcagataa catcttctga gtcataacca gcctggcagt 4920
   atgatggcct agatgcagag aaaacagctc cttggtgaat tgataagtaa aggcagaaaa 4980
   gattatatgt catacctcca ttggggaata agcataaccc tgagattctt actactgatg 5040
   agaacattat ctgcatatgc caaaaaattt taagcaaatg aaagctacca atttaaagtt 5100
   acggaatcta ccattttaaa gttaattgct tgtcaagcta taaccacaaa aataatgaat 5160
   tgatgagaaa tacaatgaag aggcaatgtc catctcaaaa tactgctttt acaaaagcag 5220
   aataaaagcg aaaagaaatg aaaatgttac actacattaa tcctggaata aaagaagccg 5280
   aaataaatga gagatgagtt gggatcaagt ggattgagga ggctgtgctg tgtgccaatg 5340
   tttcgtttgc ctcagacagg tatctcttcg ttatcagaag agttgcttca tttcatctgg 5400
   gagcagaaaa cagcaggcag ctgttaacag ataagtttaa cttgcatctg cagtattgca 5460
   tgttagggat aagtgcttat ttttaagagc tgtggagttc ttaaatatca accatggcac 5520
   tttctcctga ccccttccct aggggatttc aggattgaga aatttttcca tcgagccttt 5580
   ttaaaattgt aggacttgtt cctgtgggct tcagtgatgg gatagtacac ttcactcaga 5640
   ggcatttgca tctttaaata atttcttaaa agcctctaaa gtgatcagtg ccttgatgcc 5700
   aactaaggaa atttgtttag cattgaatct ctgaaggctc tatgaaagga atagcatgat 5760
   gtgctgttag aatcagatgt tactgctaaa atttacatgt tgtgatgtaa attgtgtaga 5820
   aaaccattaa atcattcaaa ataataaact atttttatta gagaatgtat acttttagaa 5880
   agctgtctcc ttatttaaat aaaatagtgt ttgtctgtag ttcagtgttg gggcaatctt 5940
   gggggggatt cttctctaat ctttcagaaa ctttgtctgc gaacactctt taatggacca 6000
   gatcaggatt tgagcggaag aacgaatgta actttaaggc aggaaagaca aattttattc 6060
   ttcataaagt gatgagcata taataattcc aggcacatgg caatagaggc cctctaaata 6120
   aggaataaat aacctcttag acaggtggga gattatgatc agagtaaaag gtaattacac 6180
   attttatttc cagaaagtca ggggtctata aattgacagt gattagagta atactttttc 6240
   acatttccaa agtttgcatg ttaactttaa atgcttacaa tcttagagtg gtaggcaatg 6300
   ttttacacta ttgaccttat atagggaagg gagggggtgc ctgtggggtt ttaaagaatt 6360
   ttcctttgca gaggcatttc atccttcatg aagccattca ggattttgaa ttgcatatga 6420
   gtgcttggct cttccttctg ttctagtgag tgtatgagac cttgcagtga gtttatcagc 6480
   atactcaaaa tttttttcct ggaatttgga gggatgggag gagggggtgg ggcttacttg 6540
   ttgtagcttt tttttttttt acagacttca cagagaatgc agttgtcttg acttcaggtc 6600
   tgtctgttct gttggcaagt aaatgcagta ctgttctgat cccgctgcta ttagaatgca 6660
   ttgtgaaacg actggagtat gattaaaagt tgtgttcccc aatgcttgga gtagtgattg 6720
   ttgaaggaaa aaatccagct gagtgataaa ggctgagtgt tgaggaaatt tctgcagttt 6780
   taagcagtcg tatttgtgat tgaagctgag tacattttgc tggtgtattt ttaggtaaaa 6840
   tgctttttgt tcatttctgg tggtgggagg ggactgaagc ctttagtctt ttccagatgc 6900
   aaccttaaaa tcagtgacaa gaaacattcc aaacaagcaa cagtcttcaa gaaattaaac 6960
   tggcaagtgg aaatgtttaa acagttcagt gatctttagt gcattgttta tgtgtgggtt 7020
   tctctctccc ctcccttggt cttaattctt acatgcagga acactcagca gacacacgta 7080
   tgcgaagggc cagagaagcc agacccagta agaaaaaata gcctatttac tttaaataaa 7140
   ccaaacattc cattttaaat gtggggattg ggaaccacta gttctttcag atggtattct 7200
   tcagactata gaaggagctt ccagttgaat tcaccagtgg acaaaatgag gaaaacaggt 7260
   gaacaagctt tttctgtatt tacatacaaa gtcagatcag ttatgggaca atagtattga 7320
   atagatttca gctttatgct ggagtaactg gcatgtgagc aaactgtgtt ggcgtggggg 7380
   tggaggggtg aggtgggcgc taagcctttt tttaagattt ttcaggtacc cctcactaaa 7440
   ggcaccgaag gcttaaagta ggacaaccat ggagccttcc tgtggcagga gagacaacaa 7500
   agcgctatta tcctaaggtc aagagaagtg tcagcctcac ctgattttta ttagtaatga 7560
   ggacttgcct caactccctc tttctggagt gaagcatccg aaggaatgct tgaagtaccc 7620
   ctgggcttct cttaacattt aagcaagctg tttttatagc agctcttaat aataaagccc 7680
   aaatctcaag cggtgcttga aggggaggga aagggggaaa gcgggcaacc acttttccct 7740
   agcttttcca gaagcctgtt aaaagcaagg tctccccaca agcaacttct ctgccacatc 7800
   gccaccccgt gccttttgat ctagcacaga cccttcaccc ctcacctcga tgcagccagt 7860
   agcttggatc cttgtgggca tgatccataa tcggtttcaa ggtaacgatg gtgtcgaggt 7920
   ctttggtggg ttgaactatg ttagaaaagg ccattaattt gcctgcaaat tgttaacaga 7980
   agggtattaa aaccacagct aagtagctct attataatac ttatccagtg actaaaacca 8040
   acttaaacca gtaagtggag aaataacatg ttcaagaact gtaatgctgg gtgggaacat 8100
   gtaacttgta gactggagaa gataggcatt tgagtggctg agagggcttt tgggtgggaa 8160
   tgcaaaaatt ctctgctaag actttttcag gtgaacataa cagacttggc caagctagca 8220
   tcttagcgga agctgatctc caatgctctt cagtagggtc atgaaggttt ttcttttcct 8280
   gagaaaacaa cacgtattgt tttctcaggt tttgcttttt ggcctttttc tagcttaaaa 8340
   aaaaaaaaag caaaagatgc tggtggttgg cactcctggt ttccaggacg gggttcaaat 8400
   ccctgcggcg tctttgcttt gactactaat ctgtcttcag gactctttct gtatttctcc 8460
   ttttctctgc aggtgctagt tcttggagtt ttggggaggt gggaggtaac agcacaatat 8520
   ctttgaacta tatacatcct tgatgtataa tttgtcagga gcttgacttg attgtatatt 8580
   catatttaca cgagaaccta atataactgc cttgtctttt tcaggtaata gcctgcagct 8640
   ggtgttttga gaagccctac tgctgaaaac ttaacaattt tgtgtaataa aaatggagaa 8700
   gctctaaatt gttgtggttc ttttgtgaat aaaaaaatct tgattgggga aaaaaaaa 8758

<210> 5
<211> 2534
<212> DNA
<213> Mus musculus

<220>
<223> SR-B1 mRNA

<400> 5
ggaatcccgc gccgaactcg ggggcgggct gcccgggcca tggcgcataa agcctctggc 60

cacctgcagg gctactgctg ctccggccac cgccaggcac acaccttgct gctgagggag 120

tctcggcttc tgtcatctct gtggcctccg tcacctctgt ctccgtctcc ttcaggtcct 180

gagccccgag agccccttcc gcgcacgcgg acatgggcgg cagctccagg gcgcgctggg 240

tggccttggg gttgggcgcc ctggggctgc tgtttgctgc gctcggcgtt gtcatgatcc 300

tcatggtgcc ctccctcatc aagcagcagg tgctcaagaa tgtccgcata gacccgagca 360

gcctgtcctt cgggatgtgg aaggagatcc ccgtcccttt ctacttgtct gtctacttct 420

tcgaagtggt caacccaaac gaggtcctca acggccagaa gccagtagtc cgggagcgtg 480

gaccctatgt ctacagggag ttcagacaaa aggtcaacat caccttcaat gacaacgaca 540

ccgtgtcctt cgtggagaac cgcagcctcc atttccagcc tgacaagtcg catggctcag 600

agagtgacta cattgtactg cctaacatct tggtcctggg gggctcgata ttgatggaga 660

gcaagcctgt gagcctgaag ctgatgatga ccttggcgct ggtcaccatg ggccagcgtg 720

cttttatgaa ccgcacagtt ggtgagatcc tgtggggcta tgacgatccc ttcgtgcatt 780

ttctcaacac gtacctccca gacatgcttc ccataaaggg caaatttggc ctgtttgttg 840

ggatgaacaa ctcgaattct ggggtcttca ctgtcttcac gggcgtccag aatttcagca 900

ggatccatct ggtggacaaa tggaacggac tcagcaagat cgattattgg cattcagagc 960

agtgtaacat gatcaatggg acttccgggc agatgtgggc acccttcatg acacccgaat 1020

cctcgctgga attcttcagc ccggaggcat gcaggtccat gaagctgacc tacaacgaat 1080

caagggtgtt tgaaggcatt cccacgtatc gcttcacggc ccccgatact ctgtttgcca 1140

acgggtccgt ctacccaccc aacgaaggct tctgcccatg ccgagagtct ggcattcaga 1200

atgtcagcac ctgcaggttt ggtgcgcctc tgtttctctc ccacccccac ttttacaacg 1260

ccgaccctgt gttgtcagaa gctgttcttg gtctgaaccc taacccaaag gagcattcct 1320

tgttcctaga catccatccg gtcactggga tccccatgaa ctgttctgtg aagatgcagc 1380

tgagcctcta catcaaatct gtcaagggca tcgggcaaac agggaagatc gagccagtag 1440

ttctgccgtt gctgtggttc gaacagagcg gagcaatggg tggcaagccc ctgagcacgt 1500

tctacacgca gctggtgctg atgccccagg ttcttcacta cgcgcagtat gtgctgctgg 1560

ggcttggagg cctcctgttg ctggtgccca tcatctgcca actgcgcagc caggagaaat 1620

gctttttgtt ttggagtggt agtaaaaagg gctcccagga taaggaggcc attcaggcct 1680

actctgagtc cctgatgtca ccagctgcca agggcacggt gctgcaagaa gccaagctat 1740

agggtcctga agacactata agccccccaa acctgatagc ttggtcagac cagccaccca 1800

gtccctacac cccgcttctt gaggactctc tcagcggaca gcccaccagt gccatggcct 1860

gagcccccag atgtcacacc tgtccgcacg cacggcacat ggatgcccac gcatgtgcaa 1920

aaacaactca gggaccaggg acagacctgc tgccaagtga gcctgatggg ccacaggtgt 1980

gctcttctaa atggcctgtg agccaggctg tgggaactct agctgctgtc agcccctcct 2040

gtaggagctg gccctgccca ggctcctgac ttccctcagg aagtctttct gtctttctcc 2100

atcagtctga aagccttagt tcccacagag gacggatctg tcactcctag gggctgggca 2160

tatgtcggcc tcttgtgcca aggccaggca agcagctcca ggtcctgacc agtttgcaca 2220

cacactctgg agctgtatct ggcgcttttt ctatcgtctc tgctatgtca ctgaattaac 2280

cactgtacgt ggcagaggtg gcaggcccct cagggtcctt atttttcagg catggggtca 2340

aagctagagg tatgggccgt ctacaccccc ccgccccccg gcatctagtg tacctcacca 2400

gagggtattc ggaggcccag catcctgcaa ccgacccctt ttttctactg gaagagaaat 2460

tttatcatct tttgaaagga agtcatgact gaagcaataa accttttcac tgattcaaca 2520

aaaaaaaaaa aaaa 2534

<210> 6
<211> 2759
<212> DNA
<213> Homo sapiens

<220>
<223> SR-B1 mRNA

<400> 6
gctcaggccc cgcccctgcc gccggaatcc tgaagcccaa ggctgcccgg gggcggtccg 60

gcggcgccgg cgatggggca taaaaccact ggccacctgc cgggctgctc ctgcgtgcgc 120

tgccgtcccg gatccaccgt gcctctgcgg cctgcgtgcc cggagtcccc gcctgtgtcg 180

tctctgtcgc cgtccccgtc tcctgccagg cgcggagccc tgcgagccgc gggtgggccc 240

caggcgcgca gacatgggct gctccgccaa agcgcgctgg gctgccgggg cgctgggcgt 300

cgcggggcta ctgtgcgctg tgctgggcgc tgtcatgatc gtgatggtgc cgtcgctcat 360

caagcagcag gtccttaaga acgtgcgcat cgaccccagt agcctgtcct tcaacatgtg 420

gaaggagatc cctatcccct tctatctctc cgtctacttc tttgacgtca tgaaccccag 480

cgagatcctg aagggcgaga agccgcaggt gcgggagcgc gggccctacg tgtacaggga 540

gttcaggcac aaaagcaaca tcaccttcaa caacaacgac accgtgtcct tcctcgagta 600

ccgcaccttc cagttccagc cctccaagtc ccacggctcg gagagcgact acatcgtcat 660

gcccaacatc ctggtcttgg gtgcggcggt gatgatggag aataagccca tgaccctgaa 720

gctcatcatg accttggcat tcaccaccct cggcgaacgt gccttcatga accgcactgt 780

gggtgagatc atgtggggct acaaggaccc ccttgtgaat ctcatcaaca agtactttcc 840

aggcatgttc cccttcaagg acaagttcgg attatttgct gagctcaaca actccgactc 900

tgggctcttc acggtgttca cgggggtcca gaacatcagc aggatccacc tcgtggacaa 960

gtggaacggg ctgagcaagg ttgacttctg gcattccgat cagtgcaaca tgatcaatgg 1020

aacttctggg caaatgtggc cgcccttcat gactcctgag tcctcgctgg agttctacag 1080

cccggaggcc tgccgatcca tgaagctaat gtacaaggag tcaggggtgt ttgaaggcat 1140

ccccacctat cgcttcgtgg ctcccaaaac cctgtttgcc aacgggtcca tctacccacc 1200

caacgaaggc ttctgcccgt gcctggagtc tggaattcag aacgtcagca cctgcaggtt 1260

cagtgccccc ttgtttctct cccatcctca cttcctcaac gctgacccgg ttctggcaga 1320

agcggtgact ggcctgcacc ctaaccagga ggcacactcc ttgttcctgg acatccaccc 1380

ggtcacggga atccccatga actgctctgt gaaactgcag ctgagcctct acatgaaatc 1440

tgtcgcaggc attggacaaa ctgggaagat tgagcctgtg gtcctgccgc tgctctggtt 1500

tgcagagagc ggggccatgg agggggagac tcttcacaca ttctacactc agctggtgtt 1560

gatgcccaag gtgatgcact atgcccagta cgtcctcctg gcgctgggct gcgtcctgct 1620

gctggtccct gtcatctgcc aaatccggag ccaagagaaa tgctatttat tttggagtag 1680

tagtaaaaag ggctcaaagg ataaggaggc cattcaggcc tattctgaat ccctgatgac 1740

atcagctccc aagggctctg tgctgcagga agcaaaactg tagggtcctg aggacaccgt 1800

gagccagcca ggcctggccg ctgggcctga ccggcccccc agcccctaca ccccgcttct 1860

cccggactct cccagcggac agccccccag ccccacagcc tgagcctccc agctgccatg 1920

tgcctgttgc acacctgcac acacgccctg gcacacatac acacatgcgt gcaggcttgt 1980

gcagacactc agggatggag ctgctgctga agggacttgt agggagaggc tcgtcaacaa 2040

gcactgttct ggaaccttct ctccacgtgg cccacaggcc tgaccacagg ggctgtgggt 2100

cctgcgtccc cttcctcggg tgagcctggc ctgtcccgtt cagccgttgg gcccaggctt 2160

cctcccctcc aaggtgaaac actgcagtcc cggtgtggtg gctccccatg caggacgggc 2220

caggctggga gtgccgcctt cctgtgccaa attcagtggg gactcagtgc ccaggccctg 2280

gccacgagct ttggccttgg tctacctgcc aggccaggca aagcgccttt acacaggcct 2340

cggaaaacaa tggagtgagc acaagatgcc ctgtgcagct gcccgagggt ctccgcccac 2400

cccggccgga ctttgatccc cccgaagtct tcacaggcac tgcatcgggt tgtctggcgc 2460

ccttttcctc cagcctaaac tgacatcatc ctatggactg agccggccac tctctggccg 2520

aagtggccgc aggctgtgcc cccgagctgc ccccaccccc tcacagggtc cctcagatta 2580

taggtgccca ggctgaggtg aagaggcctg ggggccctgc cttccgggcg ctcctggacc 2640

ctggggcaaa cctgtgaccc ttttctactg gaatagaaat gagttttatc atctttgaaa 2700

aataattcac tcttgaagta ataaacgttt aaaaaaatgg gaaaaaaaaa aaaaaaaaa 2759

<210> 7
<211> 2761
<212> DNA
<213> Mus musculus

<220>
<223> DMPK mRNA

<400> 7
gaactggcca gagagaccca agggatagtc agggacgggc agacatgcag ctagggttct 60

ggggcctgga caggggcagc caggccctgt gacgggaaga ccccgagctc cggcccgggg 120

aggggccatg gtgttgcctg cccaacatgt cagccgaagt gcggctgagg cagctccagc 180

agctggtgct ggacccaggc ttcctgggac tggagcccct gctcgacctt ctcctgggcg 240

tccaccagga gctgggtgcc tctcacctag cccaggacaa gtatgtggcc gacttcttgc 300

agtgggtgga gcccattgca gcaaggctta aggaggtccg actgcagagg gatgattttg 360

agattttgaa ggtgatcggg cgtggggcgt tcagcgaggt agcggtggtg aagatgaaac 420

agacggccca agtgtatgcc atgaagatta tgaataagtg ggacatgctg aagagaggcg 480

aggtgtcgtg cttccgggaa gaaagggatg tattagtgaa aggggaccgg cgctggatca 540

cacagctgca ctttgccttc caggatgaga actacctgta cctggtcatg gaatactacg 600

tgggcgggga cctgctaacg ctgctgagca agtttgggga gcggatcccc gccgagatgg 660

ctcgcttcta cctggccgag attgtcatgg ccatagactc cgtgcaccgg ctgggctacg 720

tgcacaggga catcaaacca gataacattc tgctggaccg atgtgggcac attcgcctgg 780

cagacttcgg ctcctgcctc aaactgcagc ctgatggaat ggtgaggtcg ctggtggctg 840

tgggcacccc ggactacctg tctcctgaga ttctgcaggc cgttggtgga gggcctgggg 900

caggcagcta cgggccagag tgtgactggt gggcactggg cgtgttcgcc tatgagatgt 960

tctatgggca gacccccttc tacgcggact ccacagccga gacatatgcc aagattgtgc 1020

actacaggga acacttgtcg ctgccgctgg cagacacagt tgtccccgag gaagctcagg 1080

acctcattcg tgggctgctg tgtcctgctg agataaggct aggtcgaggt ggggcaggtg 1140

atttccagaa acatcctttc ttctttggcc ttgattggga gggtctccga gacagtgtac 1200

ccccctttac accagacttc gagggtgcca cggacacatg caatttcgat gtggtggagg 1260

accggctcac tgccatggtg agcgggggcg gggagacgct gtcagacatg caggaagaca 1320

tgccccttgg ggtgcgcctg cccttcgtgg gctactccta ctgctgcatg gccttcagag 1380

acaatcaggt cccggacccc acccctatgg aactagaggc cctgcagttg cctgtgtcag 1440

acttgcaagg gcttgacttg cagcccccag tgtccccacc ggatcaagtg gctgaagagg 1500

ctgacctagt ggctgtccct gcccctgtgg ctgaggcaga gaccacggta acgctgcagc 1560

agctccagga agccctggaa gaagaggttc tcacccggca gagcctgagc cgcgagctgg 1620

aggccatccg gaccgccaac cagaacttct ccagccaact acaggaggcc gaggtccgaa 1680

accgagacct ggaggcgcat gttcggcagc tacaggaacg gatggagatg ctgcaggccc 1740

caggagccgc agccatcacg gggtccccca gtccccgggc cacggatcca ccttcccatc 1800

tagatggccc cccggccgtg gctgtgggcc agtgcccgct ggtggggcca ggccccatgc 1860

accgccgtca cctgctgctc cctgccagga tccctaggcc tggcctatcc gaggcgcgtt 1920

gcctgctcct gttcgccgct gctctggctg ctgccgccac actgggctgc actgggttgg 1980

tggcctatac cggcggtctc accccagtct ggtgtttccc gggagccacc ttcgccccct 2040

gaaccctaag actccaagcc atctttcatt taggcctcct aggaaggtcg agcgaccagg 2100

gagcgaccca aagcgtctct gtgcccatcg cgcccccccc ccccccccac cgctccgctc 2160

cacacttctg tgagcctggg tccccaccca gctccgctcc tgtgatccag gcctgccacc 2220

tggcggccgg ggagggagga acagggctcg tgcccagcac ccctggttcc tgcagagctg 2280

gtagccaccg ctgctgcagc agctgggcat tcgccgacct tgctttactc agccccgacg 2340

tggatgggca aactgctcag ctcatccgat ttcacttttt cactctccca gccatcagtt 2400

acaagccata agcatgagcc ccctatttcc agggacatcc cattcccata gtgatggatc 2460

agcaagacct ctgccagcac acacggagtc tttggcttcg gacagcctca ctcctggggg 2520

ttgctgcaac tccttccccg tgtacacgtc tgcactctaa caacggagcc acagctgcac 2580

tcccccctcc cccaaagcag tgtgggtatt tattgatctt gttatctgac tcactgacag 2640

actccgggac ccacgtttta gatgcattga gactcgacat tcctcggtat ttattgtctg 2700

tccccaccta cgacctccac tcccgaccct tgcgaataaa atacttctgg tctgccctaa 2760

a 2761

<210> 8
<211> 3243
<212> DNA
<213> Homo sapiens

<220>
<223> DMPK mRNA

<400> 8
gccacaagcc tccaccccag ctggtccccc acccaggctg cccagtttaa cattcctagt 60

cataggacct tgacttctga gaggcctgat tgtcatctgt aaataagggg taggactaaa 120

gcactcctcc tggaggactg agagatgggc tggaccggag cacttgagtc tgggatatgt 180

gaccatgcta cctttgtctc cctgtcctgt tccttccccc agccccaaat ccagggtttt 240

ccaaagtgtg gttcaagaac cacctgcatc tgaatctaga ggtactggat acaaccccac 300

gtctgggccg ttacccagga cattctacat gagaacgtgg gggtggggcc ctggctgcac 360

ctgaactgtc acctggagtc agggtggaag gtggaagaac tgggtcttat ttccttctcc 420

ccttgttctt tagggtctgt ccttctgcag actccgttac cccaccctaa ccatcctgca 480

cacccttgga gccctctggg ccaatgccct gtcccgcaaa gggcttctca ggcatctcac 540

ctctatggga gggcattttt ggcccccaga accttacacg gtgtttatgt ggggaagccc 600

ctgggaagca gacagtccta gggtgaagct gagaggcaga gagaagggga gacagacaga 660

gggtggggct ttcccccttg tctccagtgc cctttctggt gaccctcggt tcttttcccc 720

caccaccccc ccagcggagc ccatcgtggt gaggcttaag gaggtccgac tgcagaggga 780

cgacttcgag attctgaagg tgatcggacg cggggcgttc agcgaggtag cggtagtgaa 840

gatgaagcag acgggccagg tgtatgccat gaagatcatg aacaagtggg acatgctgaa 900

gaggggcgag gtgtcgtgct tccgtgagga gagggacgtg ttggtgaatg gggaccggcg 960

gtggatcacg cagctgcact tcgccttcca ggatgagaac tacctgtacc tggtcatgga 1020

gtattacgtg ggcggggacc tgctgacact gctgagcaag tttggggagc ggattccggc 1080

cgagatggcg cgcttctacc tggcggagat tgtcatggcc atagactcgg tgcaccggct 1140

tggctacgtg cacagggaca tcaaacccga caacatcctg ctggaccgct gtggccacat 1200

ccgcctggcc gacttcggct cttgcctcaa gctgcgggca gatggaacgg tgcggtcgct 1260

ggtggctgtg ggcaccccag actacctgtc ccccgagatc ctgcaggctg tgggcggtgg 1320

gcctgggaca ggcagctacg ggcccgagtg tgactggtgg gcgctgggtg tattcgccta 1380

tgaaatgttc tatgggcaga cgcccttcta cgcggattcc acggcggaga cctatggcaa 1440

gatcgtccac tacaaggagc acctctctct gccgctggtg gacgaagggg tccctgagga 1500

ggctcgagac ttcattcagc ggttgctgtg tcccccggag acacggctgg gccggggtgg 1560

agcaggcgac ttccggacac atcccttctt ctttggcctc gactgggatg gtctccggga 1620

cagcgtgccc ccctttacac cggatttcga aggtgccacc gacacatgca acttcgactt 1680

ggtggaggac gggctcactg ccatggtgag cgggggcggg gagacactgt cggacattcg 1740

ggaaggtgcg ccgctagggg tccacctgcc ttttgtgggc tactcctact cctgcatggc 1800

cctcagggac agtgaggtcc caggccccac acccatggaa ctggaggccg agcagctgct 1860

tgagccacac gtgcaagcgc ccagcctgga gccctcggtg tccccacagg atgaaacagc 1920

tgaagtggca gttccagcgg ctgtccctgc ggcagaggct gaggccgagg tgacgctgcg 1980

ggagctccag gaagccctgg aggaggaggt gctcacccgg cagagcctga gccgggagat 2040

ggaggccatc cgcacggaca accagaactt cgccagtcaa ctacgcgagg cagaggctcg 2100

gaaccgggac ctagaggcac acgtccggca gttgcaggag cggatggagt tgctgcaggc 2160

agagggagcc acagctgtca cgggggtccc cagtccccgg gccacggatc caccttccca 2220

tctagatggc cccccggccg tggctgtggg ccagtgcccg ctggtggggc caggccccat 2280

gcaccgccgc cacctgctgc tccctgccag ggtccctagg cctggcctat cggaggcgct 2340

ttccctgctc ctgttcgccg ttgttctgtc tcgtgccgcc gccctgggct gcattgggtt 2400

ggtggcccac gccggccaac tcaccgcagt ctggcgccgc ccaggagccg cccgcgctcc 2460

ctgaacccta gaactgtctt cgactccggg gccccgttgg aagactgagt gcccggggca 2520

cggcacagaa gccgcgccca ccgcctgcca gttcacaacc gctccgagcg tgggtctccg 2580

cccagctcca gtcctgtgat ccgggcccgc cccctagcgg ccggggaggg aggggccggg 2640

tccgcggccg gcgaacgggg ctcgaagggt ccttgtagcc gggaatgctg ctgctgctgc 2700

tgctgctgct gctgctgctg ctgctgctgc tgctgctgct gctgctgggg ggatcacaga 2760

ccatttcttt ctttcggcca ggctgaggcc ctgacgtgga tgggcaaact gcaggcctgg 2820

gaaggcagca agccgggccg tccgtgttcc atcctccacg cacccccacc tatcgttggt 2880

tcgcaaagtg caaagctttc ttgtgcatga cgccctgctc tggggagcgt ctggcgcgat 2940

ctctgcctgc ttactcggga aatttgcttt tgccaaaccc gctttttcgg ggatcccgcg 3000

cccccctcct cacttgcgct gctctcggag ccccagccgg ctccgcccgc ttcggcggtt 3060

tggatattta ttgacctcgt cctccgactc gctgacaggc tacaggaccc ccaacaaccc 3120

caatccacgt tttggatgca ctgagacccc gacattcctc ggtatttatt gtctgtcccc 3180

acctaggacc cccacccccg accctcgcga ataaaaggcc ctccatctgc ccaaagctct 3240

gga 3243

## Description

### Technical Field

The present invention relates to e.g., a nucleic acid complex or a salt thereof that can produce an antisense effect in the nervous system, for example, the central nervous system, and a composition comprising the same.

### Background Art

In recent years, an oligonucleotide has been drawing attention in the ongoing development of a pharmaceutical called a nucleic acid medicine, and in particular, development of a nucleic acid medicine utilizing the antisense method is actively carried out from the viewpoint of high selectivity on the target gene and low toxicity. The antisense method is a method comprising selectively modifying or inhibiting the expression of a protein encoded by a target gene or the activity of miRNA by introducing into a cell an oligonucleotide complementary to a target sense strand that is a partial sequence of mRNA or miRNA transcribed from a target gene (antisense oligonucleotide, herein often referred to as "ASO").

Patent Literature 1 discloses a double-stranded nucleic acid molecule which consists of a first oligomeric compound, and a second oligomeric compound comprising a conjugated group such as cholesterol, and can regulate the amount or activity of a target nucleic acid in an extrahepatic tissue or an extrahepatic cell, or in a hepatic tissue or a hepatocyte, and an antisense compound consisting of the double-stranded nucleic acid molecule.

Meanwhile, in order to produce an antisense effect in the central nervous system including the brain, it is necessary to deliver the nucleic acid agent such as the aforementioned ASO to the central nervous system. However, in the brain there are mechanisms called a blood-brain barrier (hereinafter often referred to as the "BBB"), a blood-cerebrospinal fluid barrier (hereinafter often referred to as the "BCSFB"), a cerebrospinal fluid-brain barrier (hereinafter often referred to as the "CSFBB"), and the like that select and restrict substances that transition to the brain via the blood or the cerebrospinal fluid. While the BBB mechanism, the BCSFB mechanism and/or the CSFBB mechanism and so on protect the brain from toxic substances, these also serve as barriers for drug delivery to the brain. Therefore, a method of delivering a nucleic acid agent, such as an ASO, to the central nervous system including the brain, is required.

### Citation List

### Patent Literature

Patent Literature 1: WO 2017/053999

### Summary of Invention

### Technical Problem

An object of the present invention is to provide a nucleic acid agent that is efficiently delivered to the nervous system, for example, to the central nervous system to which drug delivery can be prevented by the BBB mechanism, BCSFB mechanism and/or CSFBB mechanism and so on (herein often collectively abbreviated as "BBB and so on"), and produces an antisense effect on a target transcriptional product at the delivery site, and a composition comprising the same.

### Solution to Problem

To solve the above-described problem, the present inventors studied intensively and found that a nucleic acid complex formed by annealing an ASO and a complementary strand of the ASO bound to a C₂₂₋₃₅ alkyl group optionally substituted with a hydroxy group, or an analog thereof is efficiently delivered to the central nervous system and exhibit a high antisense effect. Based on these findings, the inventors have completed the present invention. The present invention thus encompasses the following aspects.
(1) A nucleic acid complex comprising a first nucleic acid strand and a second nucleic acid strand or a salt thereof, wherein:
   the first nucleic acid strand comprises a base sequence capable of hybridizing to at least a part of a target transcriptional product, and has an antisense effect on the target transcriptional product;
   the second nucleic acid strand comprises a base sequence complementary to the first nucleic acid strand, and is bound to a C₂₂₋₃₅ alkyl group optionally substituted with a hydroxy group or an analog thereof; and
   the first nucleic acid strand is annealed to the second nucleic acid strand.
   (herein often collectively abbreviated as the "nucleic acid complex of the present inventi on".)
(2) A first nucleic acid strand bound to a C₂₂₋₃₅ alkyl group optionally substituted with a hydroxy group or an analog thereof, or a salt thereof, comprising a base sequence capable of hybridizing to at least a part of a target transcriptional product, wherein said first nucleic acid strand has an antisense effect on the target transcriptional product.
(3) The nucleic acid complex, nucleic acid strand, or a salt thereof according to (1) or (2), wherein the second nucleic acid strand or the first nucleic acid strand is bound to a C₂₂₋₃₅ alkyl group optionally substituted with a hydroxy group or an analog thereof, via a linker represented by Formula I. 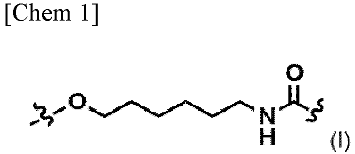
(4) The nucleic acid complex, nucleic acid strand, or a salt thereof according to any of (1) to (3), wherein the C₂₂₋₃₅ alkyl group optionally substituted with a hydroxy group, or an analog thereof is an unsubstituted linear C₂₂₋₂₄ or linear C₂₆ alkyl group.
(5) The nucleic acid complex, nucleic acid strand, or a salt thereof according to any of (1) to (4), wherein said first nucleic acid strand comprises at least four contiguous deoxyribonucleosides.
(6) The nucleic acid complex, nucleic acid strand, or a salt thereof according to any of (1) to (5), wherein said first nucleic acid strand is a gapmer.
(7) The nucleic acid complex, nucleic acid strand, or a salt thereof according to any of (1) to (6), wherein said second nucleic acid strand comprises at least four contiguous ribonucleosides complementary to at least four contiguous deoxyribonucleosides in said first nucleic acid strand.
(8) The nucleic acid complex, nucleic acid strand, or a salt thereof according to any of (1) to (4), wherein said first nucleic acid strand is a mixmer.
(9) The nucleic acid complex, nucleic acid strand, or a salt thereof according to any of (1) to (8), wherein said first nucleic acid strand is 13 to 20 bases in length.
(10) The nucleic acid complex, nucleic acid strand, or a salt thereof according to any of (1) to (9), wherein said second nucleic acid strand does not comprise a natural ribonucleoside.
(11) The nucleic acid complex, nucleic acid strand, or a salt thereof according to any of (1) to (10), wherein the nucleic acid portion of said second nucleic acid strand consists of deoxyribonucleosides and/or sugar-modified nucleosides linked by a modified or unmodified internucleoside bond.
(12) A composition for regulating expression or editing of a target transcriptional product in the central nervous system, comprising the nucleic acid complex, nucleic acid strand, or a salt thereof according to any of (1) to (11).
(13) The composition according to (12), for treating a central nervous system disease.
(14) A composition for delivering a drug to the central nervous system, comprising the nucleic acid complex, nucleic acid strand, or a salt thereof according to any of (1) to (11).
(15) The composition according to any of (12) to (14), wherein said central nervous system is selected from the group consisting of cerebral cortex, basal ganglion, cerebral white matter, diencephalon, brainstem, cerebellum, and spinal cord.
(16) The composition according to any of (12) to (14), wherein said central nervous system is selected from the group consisting of frontal lobe, temporal lobe, hippocampus, parahippocampal gyrus, parietal lobe, occipital lobe, striatum, globus pallidus, claustrum, thalamus, subthalamic nucleus, midbrain, substantia nigra, pons, medulla oblongata, cerebellar cortex, cerebellar nucleus, cervical spinal cord, thoracic spinal cord, and lumbar spinal cord.
(17) The composition according to any of (12) to (16), for intravenous administration or subcutaneous administration.
(18) The composition according to any of (12) to (17), comprising 5 mg/kg or more of said nucleic acid complex, nucleic acid strand, or a salt thereof in a single dose.
(19) The composition according to any of (12) to (18), wherein said nucleic acid complex, nucleic acid strand, or a salt thereof transitions from blood to the brain (brain parenchyma).

The present description encompasses the disclosures in Japanese Patent Application No. 2019-168930, which forms the basis for the priority of the present application.

### Advantageous Effects of Invention

The present invention can provide a nucleic acid agent that is efficiently delivered to the central nervous system and produces an antisense effect at the delivery site, and a composition comprising the same.

### Brief Description of Drawings

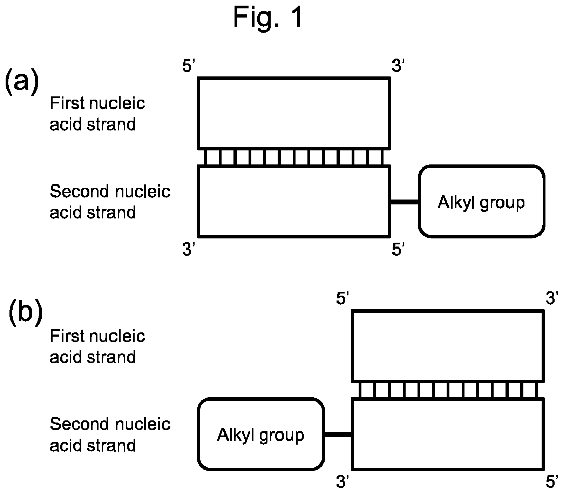

[Figure 1] Figure 1 is a schematic diagram showing examples of specific embodiments of the nucleic acid complex used in the present invention. This figure shows two modes in terms of a binding position of a C₂₂₋₃₅ alkyl group optionally substituted with a hydroxy group or an analog thereof (simply designated as "alkyl group" in the figure) in the second nucleic acid strand. Figure 1a shows a nucleic acid complex in which a C₂₂₋₃₅ alkyl group optionally substituted with a hydroxy group or an analog thereof is bound to the 5' end of the second nucleic acid strand. Figure 1b shows a nucleic acid complex in which a C₂₂₋₃₅ alkyl group optionally substituted with a hydroxy group or an analog thereof is bound to the 3' end of the second nucleic acid strand.
[Figure 2] Figure 2 is a diagram showing an example of a general mechanism of the antisense method. In the diagram "X" indicates the position of suppression or inhibition in the steps from expression of a gene to translation. The figure inside the dashed line is a schematic diagram in which a heteroduplex portion is recognized by RNase H and the mRNA of the target gene is degraded.
[Figure 3] Figure 3 is a diagram showing the structures of various bridged nucleic acids.
[Figure 4] Figure 4 is a diagram showing the structures of various natural nucleotides or non-natural nucleotides.

### Description of Embodiments

### <Nucleic acid complex>

The first aspect of the present invention is a nucleic acid complex. The present nucleic acid complex can transition from blood to the brain (brain parenchyma), for example, can cross BBB and so on. The present nucleic acid complex comprises a first nucleic acid strand and a second nucleic acid strand. The second nucleic acid strand is a nucleotide strand that comprises a base sequence complementary to the first nucleic acid strand. In the nucleic acid complex, the first nucleic acid strand is annealed to the second nucleic acid strand. In an embodiment, the second nucleic acid strand is bound to a C₂₂₋₃₅ alkyl group optionally substituted with a hydroxy group, or an analog thereof.

A representative schematic diagram of the nucleic acid complex is shown in Figure 1. Figure 1a shows a nucleic acid complex in which a C₂₂₋₃₅ alkyl group optionally substituted with a hydroxy group or an analog thereof is bound to the 5' end of the second nucleic acid strand. Figure 1b shows a nucleic acid complex in which a C₂₂₋₃₅ alkyl group optionally substituted with a hydroxy group or an analog thereof is bound to the 3' end of the second nucleic acid strand. However, as described below, the C₂₂₋₃₅ alkyl group optionally substituted with a hydroxy group, or the analog thereof may be bound to the 5' end, the 3' end, or both the ends of the second nucleic acid strand, or to an interior nucleotide in the second nucleic acid strand.

In an embodiment, the first nucleic acid strand is a nucleotide strand comprising a base sequence capable of hybridizing to at least a part of a target transcriptional product. In a certain embodiment, the first nucleic acid strand is a nucleotide strand having an antisense effect on a transcriptional product of a target gene, or a target transcriptional product.

In an embodiment, the present invention relates to a single-stranded nucleic acid strand bound to a C₂₂₋₃₅ alkyl group optionally substituted with a hydroxy group, or an analog thereof. The present nucleic acid strand can transition from blood to the brain (brain parenchyma), for example, can cross BBB and so on. This nucleic acid strand corresponds to the first nucleic acid strand of aforementioned nucleic acid complex, and is a nucleotide strand comprising a base sequence capable of hybridizing to at least a part of a target transcriptional product. In a certain embodiment, the nucleic acid strand is a nucleotide strand having an antisense effect on a transcriptional product of a target gene, or a target transcriptional product.

### (Definition of terms)

A "target transcriptional product" means herein any RNA that can be a target of a nucleic acid complex of the present invention, and is synthesized by a DNA-dependent RNA polymerase. In general, a transcriptional product of a target gene corresponds to the same. Specifically, the same may comprise mRNA transcribed from a target gene (such as mature mRNA, mRNA precursor, and mRNA without a base modification), and non-coding RNA (ncRNA) such as miRNA.

A "target gene" is not particularly limited herein, and examples thereof include a gene derived from an organism into which a nucleic acid complex of the present invention is introduced, such as a gene whose expression is increased in various diseases. Further, a target transcriptional product may comprise mRNA transcribed from genomic DNA encoding a target gene, and further mRNA without a base modification, and an unprocessed mRNA precursor and so on. A "target transcriptional product" may comprise not only mRNA, but also non-coding RNA (ncRNA) such as miRNA. Further, a "transcriptional product" may be, in general, any RNA synthesized by a DNA-dependent RNA polymerase. In an embodiment, a "target transcriptional product" may comprise, for example, a metastasis associated lung adenocarcinoma transcript 1 (herein often denoted as "Malat1"), a scavenger receptor B1 (herein often denoted as "SR-B 1 mRNA"), a myotonic dystrophy protein kinase (herein often denoted as "DMPK"), transthyretin (herein often denoted as "TTR"), or a gene of Apolipoprotein B (herein often denoted as "ApoB"), for example, non-coding RNA or mRNA thereof. The base sequence of murine Malat1 non-coding RNA is shown in SEQ ID NO: 3, and the base sequence of human Malat1 non-coding RNA is shown in SEQ ID NO: 4. Also, the base sequence of murine SR-B1 mRNA is shown in SEQ ID NO: 5, and the base sequence of human SR-B 1 mRNA is shown in SEQ ID NO: 6. Further, the base sequence of murine DMPK mRNA is shown in SEQ ID NO: 7, and the base sequence of human DMPK mRNA is shown in SEQ ID NO:8. In each of SEQ ID NO: 3 to 8, the base sequence of mRNA is replaced with the base sequence of DNA. Information on the base sequences of these genes and transcriptional products is available from public databases, such as the NCBI (U.S. National Center for Biotechnology Information) database.

An "antisense oligonucleotide (ASO)" or an "antisense nucleic acid" refers herein to a single-stranded oligonucleotide comprising a complementary base sequence that is capable of hybridizing to at least a part, e.g., any target region, of a target transcriptional product, and can inhibit and regulate the expression of a transcriptional product of the target gene, or the level of the target transcriptional product through an antisense effect. In a nucleic acid complex of the present invention, the first nucleic acid strand functions as an ASO, and the target region thereof may comprise a 3'UTR, a 5'UTR, an exon, an intron, a coding region, a translation initiation region, a translation termination region, or any other nucleic acid region. The target region of a target transcriptional product may be at least 8 bases in length, for example, from 10 to 35 bases in length, from 12 to 25 bases in length, from 13 to 20 bases in length, from 14 to 19 bases in length, or from 15 to 18 bases in length.

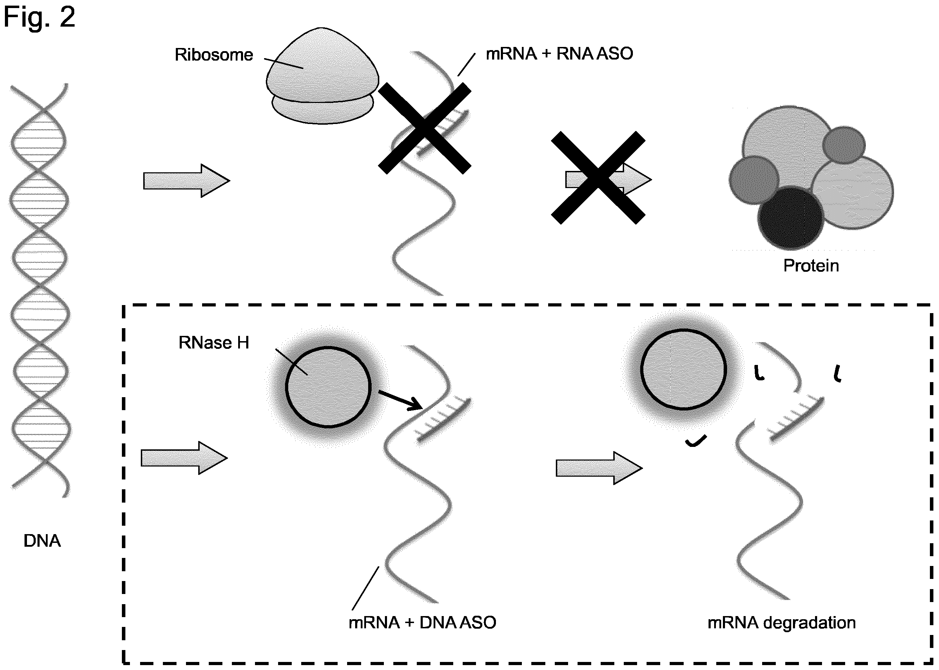

An "antisense effect" refers to an effect of regulating the expression or editing to be produced on a target transcriptional product by hybridization of an ASO to the target transcriptional product (e.g., RNA-sense strand). To "regulate the expression or editing of a target transcriptional product" means an effect of inhibiting or decreasing the expression of a target gene or the expression amount of a target transcriptional product (the "expression amount of a target transcriptional product" is herein often denoted as a "level of a target transcriptional product"), inhibiting translation, modifying a splicing function (such as exon skipping), or degrading a transcriptional product. For example, as illustrated in Figure 2, regarding inhibition of translation, when an oligonucleotide (e.g., RNA) is introduced into a cell as an ASO, the ASO binds to mRNA or the like that is a transcriptional product of a target gene to form a partial double strand. This partial double strand serves as a cover to prevent translation by ribosomes, and as a result expression of a protein encoded by the target gene is inhibited at the translational level (Figure 2, symbol X outside the dashed line). Meanwhile, when oligonucleotides comprising DNA are introduced into a cell as an ASO, a partial DNA-RNA heteroduplex strand is formed. As a result of this heteroduplex strand structure being recognized by RNase H, the mRNA of the target gene is degraded and expression of a protein encoded by the target gene inhibited at the expression level (Figure 2, inside the dashed line). This is referred to as "RNase H dependent pathway". Furthermore, in a certain example, an antisense effect can be brought about by targeting an intron of a mRNA precursor. An antisense effect may be brought about by targeting a miRNA, and in this case the function of the miRNA is inhibited and the expression of the gene whose expression is normally regulated by the miRNA may be increased. In an embodiment, the expression regulation of a target transcriptional product may be a decrease in the amount of the target transcriptional product.

The term "nucleic acid" or "nucleic acid molecule" as used herein means a nucleoside or a nucleotide in the case of a monomer, an oligonucleotide in the case of an oligomer, and a polynucleotide in the case of a polymer.

A "nucleoside" generally refers to a molecule consisting of a combination of a base and a sugar. The sugar moiety of a nucleoside is usually, but without limitation, composed of a pentofuranosyl sugar, and specific examples thereof include ribose and deoxyribose. The base moiety (nucleobase) of a nucleoside is usually a heterocyclic base moiety. Examples thereof include, but not limited to, adenine, cytosine, guanine, thymine, uracil, and other modified nucleobases (modified bases).

A "nucleotide" refers to a molecule in which a phosphate group is covalently bonded to the sugar moiety of the nucleoside. In the case of a nucleotide comprising a pentofuranosyl sugar, a phosphate group is usually linked to the hydroxyl group at the 2', 3', or 5' position of the sugar.

An "oligonucleotide" means a linear oligomer formed by linking several to dozens of contiguous nucleotides through covalent bonds between hydroxyl groups in the sugar moiety and phosphate groups. A "polynucleotide" means a linear polymer formed by linking a larger number of nucleotides than in an oligonucleotide, namely dozens or more, and preferably hundreds or more nucleotides through the aforedescribed covalent bonds. Inside the structure of an oligonucleotide or a polynucleotide, a phosphate group is generally considered to form an internucleoside bond.

The term "nucleic acid strand" or simply "strand" as used herein means an oligonucleotide or a polynucleotide. A nucleic acid strand can be produced as a full-length strand, or a partial strand by a chemical synthesis method, for example, using an automated synthesizer, or by an enzymatic process by a polymerase, a ligase, or a restriction reaction. A nucleic acid strand may comprise a natural nucleotide and/or a non-natural nucleotide.

A "natural nucleoside" means herein a naturally occurring nucleoside. Examples thereof include, for example, a ribonucleoside consisting of ribose and a base such as adenine, cytosine, guanine, or uracil, and a deoxyribonucleoside consisting of deoxyribose and the aforedescribed base such as adenine, cytosine, guanine, or thymine. A ribonucleoside found in RNA, and a deoxyribonucleoside found in DNA may be respectively referred to as "DNA nucleoside" and "RNA nucleoside". Similarly, a "natural nucleotide" is a naturally occurring nucleotide that is a molecule in which a phosphate group is covalently bonded to the sugar moiety of a natural nucleoside. Examples thereof include a ribonucleotide which is known as a constituent of RNA, and in which a phosphate group is bound to a ribonucleoside, and a deoxyribonucleotide which is known as a constituent of DNA, and in which a phosphate group is bound to a deoxyribonucleoside.

A "non-natural nucleoside" refers herein to any nucleoside other than natural nucleosides. Examples thereof include a modified nucleoside, or a nucleoside mimic. A "modified nucleoside" means herein a nucleoside having a modified sugar moiety and/or a modified nucleobase. A nucleic acid strand comprising a non-natural oligonucleotide is in many cases more preferable than a natural type, because of such desirable characteristics as enhanced cellular uptake, enhanced affinity for a nucleic acid target, increased stability in the presence of a nuclease, or increased inhibitory activity.

A "mimic" refers herein to a functional group that replaces a sugar, a nucleobase, and/or an internucleoside bond. In general, a mimic is used in place of a sugar or a combination of sugar-internucleoside bond, and a nucleobase is maintained for hybridization to a selected target. A "nucleoside mimic" comprises a structure used for replacing a sugar, or replacing a sugar and a base, or replacing a bond and so on between monomeric subunits constituting an oligomeric compound, at one or more positions of an oligomeric compound. An "oligomeric compound" means a polymer of linked monomeric subunits capable of hybridizing to at least some region of a nucleic acid molecule. Example of a nucleoside mimic include morpholino, cyclohexenyl, cyclohexyl, tetrahydropyranyl, bicyclic or tricyclic sugar mimic, e.g., a nucleoside mimic having a non-furanose sugar unit.

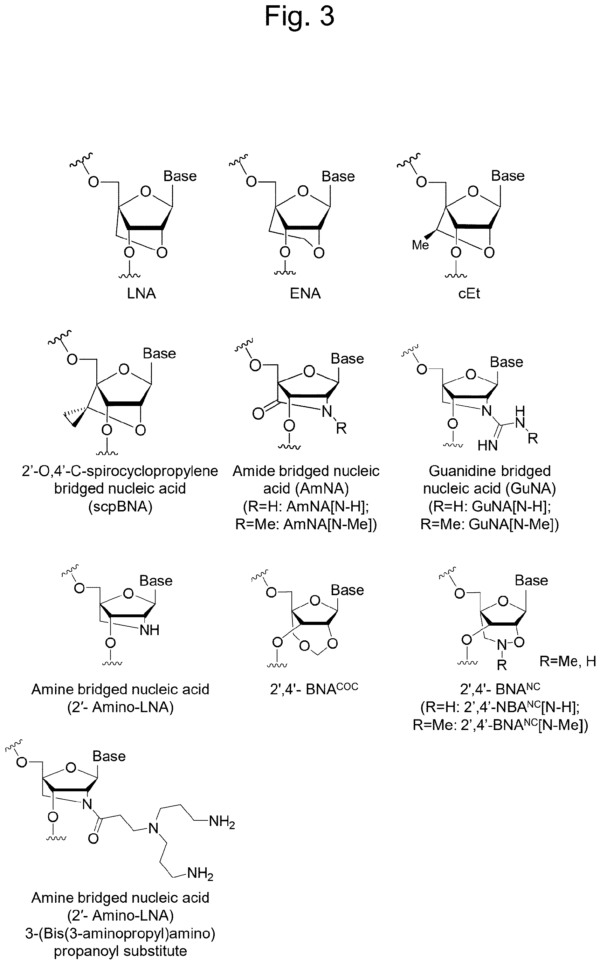

A "bicyclic nucleoside" means herein a modified nucleoside comprising a bicyclic sugar moiety. A nucleic acid comprising a bicyclic sugar moiety is generally referred to as bridged nucleic acid (BNA). A nucleoside comprising a bicyclic sugar moiety is herein sometimes referred to as "bridged nucleoside". Examples of a bridged nucleic acid are partly illustrated in Figure 3.

A bicyclic sugar may be a sugar in which the carbon atom at the 2' position and the carbon atom at the 4' position are bridged via two or more atoms. Examples of a bicyclic sugar are publicly known to those skilled in the art. A subgroup of nucleic acid (BNA) comprising a bicyclic sugar may be described as having a carbon atom at the 2' position and a carbon atom at the 4' position bridged by 4'-(CH₂)ₚ-O-2', 4'-(CH₂)ₚ-CH₂-2', 4'-(CH₂)ₚ-S-2', 4'-(CH₂)ₚ-O CH₂O-2', 4'-(CH₂)ₙ-N(R₃)-O-(CH₂)ₘ-2' [wherein p, m, and n respectively represent integers of 1 to 4, 0 to 2, and 1 to 3; and R₃ represents a hydrogen atom, an alkyl group, an alkenyl group, a cycloalkyl group, an aryl group, an aralkyl group, an acyl group, a sulfonyl group, or a unit substituent (such as a fluorescent or chemiluminescent labeling molecule, a functional group having nucleic acid cleavage activity, and intracellular or intranuclear localization signal peptide)]. Further, in the BNA according to a specific embodiment, with respect to an OR₂ substituent on the carbon atom at the 3' position, and an OR₁ substituent on the carbon atom at the 5' position, R₁ and R₂ are typically a hydrogen atom, but they may be the same or different from each other, and, further, a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, a phosphate group protected by a protecting group for nucleic acid synthesis, or P(R₄)R₅ [wherein R₄ and R₅ may be the same or different from each other, and respectively represent a hydroxyl group, a hydroxyl group protected by a protecting group for nucleic acid synthesis, a mercapto group, a mercapto group protected by a protecting group for nucleic acid synthesis, an amino group, an alkoxy group having 1 to 5 carbon atoms, an alkylthio group having 1 to 5 carbon atoms, a cyanoalkoxy group having 1 to 6 carbon atoms, or an amino group substituted with an alkyl group having 1 to 5 carbon atoms]. Non-restrictive examples of such BNA include methyleneoxy (4'-CH₂-O-2') BNA (LNA (Locked Nucleic Acid^{®}, also known as 2',4'-BNA), e.g., α-L-methyleneoxy (4'-CH₂-O-2') BNA, or β-D-methyleneoxy (4'-CH₂-O-2') BNA, ethyleneoxy (4'-(CH₂)₂-O-2') BNA (also known as ENA), β-D-thio (4'-CH₂-S-2') BNA, aminooxy (4'-CH₂-O-N(R₃)-2') BNA, oxyamino(4'-CH₂-N(R)-O-2') BNA (e.g., R=H, Me) (also known as 2',4'-BNA^{NC} (e.g., R=H is 2',4'-BNA^{NC}[N-H], R=Me is 2',4'-BNA^{NC}[N-Me])), 2',4'-BNA^{coc}, 3'-amino-2',4'-BNA, 5'-methyl BNA, (4'-CH(CH₃)-O-2') BNA (also known as cEt BNA), (4'-CH(CH₂OCH₃)-O-2') BNA (also known as cMOE BNA), amide bridged nucleic acid (4'-C(O)-N(R)-2') BNA (e.g., R=H, Me) (also known as AmNA (e.g., R=H is AmNA[N-H], R=Me is AmNA [N-Me])), guanidine bridged nucleic acid (also known as GuNA (e.g., in Figure 3, R=H is GuNA[N-H] and R=Me is GuNA[N-Me])), 2'-0,4'-C-spirocyclopropylene bridged nucleic acid (also known as scpBNA), amine bridged nucleic acid (also known as 2'-Amino-LNA) (e.g., 3-(Bis(3-aminopropyl)amino)propanoyl substitute), and other BNAs publicly known to those skilled in the art. A bicyclic nucleoside having a methyleneoxy (4'-CH₂-O-2') bridge is herein sometimes referred to as "LNA nucleoside".

A "cationic nucleoside" is herein a modified nucleoside present in a cationic form rather than in a neutral form (e.g., a neutral form of a ribonucleoside) at a certain pH (e.g., a human physiological pH (about 7.4), or a pH at the delivered site (e.g., an organelle, a cell, a tissue, an organ, or an organism)). The cationic nucleoside may comprise one or more cationically modified groups at any position of the nucleoside. In an embodiment, the cationic nucleoside is, for example, amine bridged nucleic acid (also known as 2'-Amino-LNA) (e.g., 3-(Bis(3-aminopropyl)amino)propanoyl substitute), aminoalkyl-modified nucleic acid (e.g., 2'-O-methyl or 4'-CH₂CH₂CH₂NH₂ substitute), or guanidine bridged nucleic acid (GuNA (e.g., in Figure 3, R=H is GuNA[N-H] and R=Me is GuNA[N-Me]).

A "non-natural nucleotide" refers herein to any nucleotide other than natural nucleotides and comprises a modified nucleotide and a nucleotide mimic. A "modified nucleotide" means herein a nucleotide having any one or more of a modified sugar moiety, a modified internucleoside bond, and a modified nucleobase.

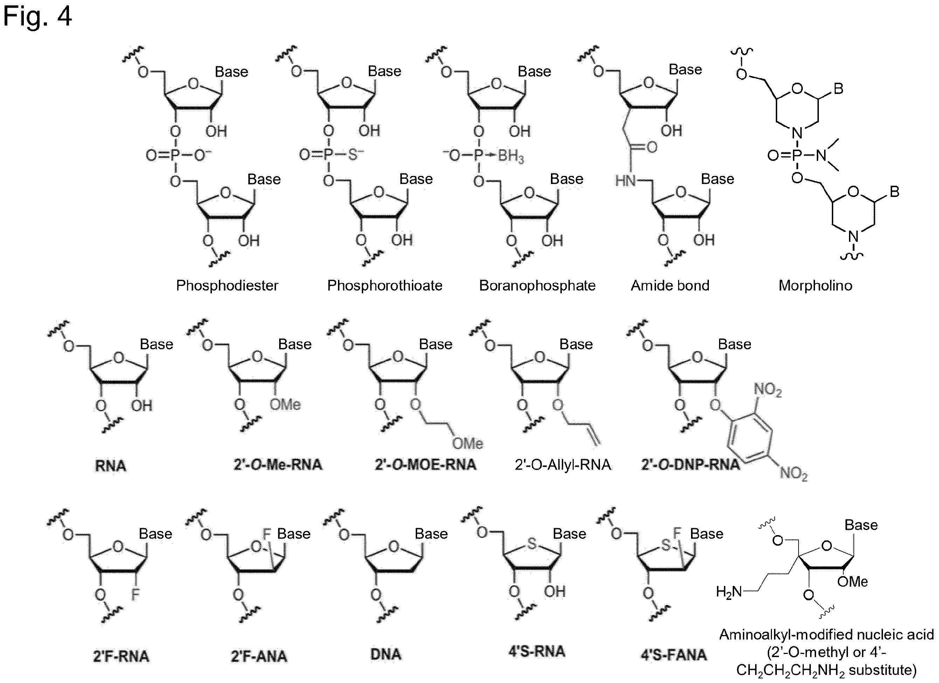

A "nucleotide mimic" comprises a structure used for replacing a nucleoside and a bond at one or more positions of an oligomeric compound. Examples of a nucleotide mimic include a peptide nucleic acid, and a morpholino nucleic acid (a morpholino linked by -N(H)-C(=O)-O- or other non-phosphodiester bonds). A peptide nucleic acid (PNA) is a nucleotide mimic having a main chain in which N-(2-aminoethyl)glycine is linked by an amide bond in place of a sugar. An example of the structure of a morpholino nucleic acid is shown in Figure 4. A nucleic acid strand comprising a non-natural oligonucleotide herein has in many cases such desirable characteristics as enhanced cellular uptake, enhanced affinity for a nucleic acid target, increased stability in the presence of a nuclease, or increased inhibitory activity. Therefore, it is more preferable than a natural nucleotide.

A "modified internucleoside bond" means herein an internucleoside bond having a substitution or any change from a naturally occurring internucleoside bond (i.e., phosphodiester bond). A modified internucleoside bond comprises a phosphorus-containing internucleoside bond comprising a phosphorus atom, and a phosphorus-free internucleoside bond not comprising a phosphorus atom. Examples of a typical phosphorus-containing internucleoside bond include, but not limited to, a phosphodiester bond, a phosphorothioate bond, a phosphorodithioate bond, a phosphotriester bond (a methylphosphotriester bond and a ethylphosphotriester bond described in US Patent No. 5,955,599), an alkylphosphonate bond (e.g., a methylphosphonate bond described in US Patent Nos. 5,264,423 and 5,286,717, and a methoxypropylphosphonate bond described in WO 2015/168172), an alkylthiophosphonate bond, a boranophosphate bond, an internucleoside bond containing a cyclic guanidine moiety (e.g., the following structure) 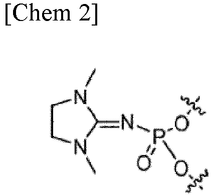 , an internucleoside bond for use in self-neutralizing nucleic acid (ZON) described in WO 2016/081600, and a phosphoramidate bond. A phosphorothioate bond is an internucleoside bond in which a non-bridging oxygen atom of a phosphodiester bond is substituted with a sulfur atom. A method for preparing a phosphorus-containing bond or a phosphorus-free bond is well known. A modified internucleoside bond should preferably be a bond whose nuclease resistance is higher than a naturally occurring internucleoside bond.

When an internucleoside bond has a chiral center, the internucleoside bond may be chirally controlled. The term "chirally controlled" means that a single diastereomer is present with respect to the chiral center, for example, chirally bound phosphorus. An internucleoside bond chirally controlled may be completely chirally pure, or may have a high chiral purity, for example, a chiral purity of 90% de, 95% de, 98% de, 99% de, 99.5% de, 99.8% de, 99.9% de, or more. A "chiral purity" herein refers to the proportion of one diastereomer in a diastereomer mixture, is represented as the diastereomeric excess rate (% de), and is defined as (diastereomer of interest - Other diastereomers)/(Total diastereomer) × 100(%).

An internucleoside bond may be, for example, a phosphorothioate bond chirally controlled to an Rp configuration or Sp configuration, or an internucleoside bond containing a cyclic guanidine moiety (e.g., the following structure). 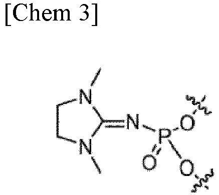 A method for preparing an internucleoside bond chirally controlled is publicly known, and a phosphorothioate bond chirally controlled to an Rp configuration or Sp configuration can be synthesized according to, for example, a method described in Naoki Iwamoto et al., Angew. Chem. Int. Ed. Engl. 2009, 48(3), 496-9, Natsuhisa Oka et al., J. Am. Chem. Soc. 2003, 125, 8307-8317, Natsuhisa Oka et al., J. Am. Chem. Soc. 2008, 130, 16031-16037, Yohei Nukaga et al., J. Org. Chem. 2016, 81, 2753-2762, or Yohei Nukaga et al., J. Org. Chem. 2012, 77, 7913-7922. A phosphorothioate bond chirally controlled to an Rp configuration or Sp configuration is also publicly known, and is known to produce an effect described in, for example, Naoki Iwamoto et al., Nat. Biotechnol,. 2017, 35(9), 845-851, or Anastasia Khvorova et al., Nat. Biotechnol., 2017, 35(3), 238-248.

A "modified nucleobase" or a "modified base" means herein any nucleobases other than adenine, cytosine, guanine, thymine, and uracil. Examples of a modified nucleobase include, but not limited to, 5-methylcytosine, 5-fluorocytosine, 5-bromocytosine, 5-iodocytosine, N4-methylcytosine, N6-methyladenine, 8-bromoadenine, N2-methylguanine, and 8-bromoguanine. A preferable modified nucleobase is 5-methylcytosine. An "unmodified nucleobase" or an "unmodified base" is synonymous with a natural nucleobase, and means adenine (A) and guanine (G), which are purine bases, as well as thymine (T), cytosine (C), and uracil (U), which are pyrimidine bases.

The term "modified sugar" refers herein to a sugar having a substitution and/or any change from a natural sugar moiety (i.e., a sugar moiety found in DNA(2'-H) or RNA(2'-OH)). A nucleic acid strand may herein, in some cases, comprise one or more modified nucleosides including a modified sugar. A sugar-modified nucleoside can confer beneficial biological properties, such as an enhanced nuclease stability, an increased binding affinity, or the like to a nucleic acid strand. A nucleoside may comprise a chemically modified ribofuranose ring moiety. Examples of a chemically modified ribofuranose ring include, but not limited to, addition of a substituent (including 5' or 2' substituent), formation of a bicyclic nucleic acid (bridged nucleic acid, BNA, LNA, ENA, AmNA(AmNA[N-H], AmNA[N-Me]), GuNA(GuNA[N-H], GuNA[N-Me]), scpBNA, 2',4'-BNA^{NC}(2',4'-BNA^{NC}[N-H], 2',4'-BNA^{NC}[N-Me]), 2',4'-BNA^{coc} and so on) by forming a bridge between non-geminal ring atoms, substitution of a ribosyl ring oxygen atom with S, N(R), or C(R1)(R2) (wherein each of R, R1 and R2 independently represents H, a C₁ to C₁₂ alkyl, or a protective group), and a combination thereof. Herein, examples of a nucleoside having a modified sugar moiety include, but not limited to, a nucleoside comprising a substituent of 5'-vinyl, 5'-methyl(R or S), 4'-S, 2'-F(2'-fluoro group), 2'-OCH₃ (2'-OMe group or 2'-O-methyl group), and 2'-O(CH₂)₂OCH₃. A substituent at the 2' position may be selected from allyl, amino, azide, thio, -O-allyl, -O-C₁-C₁₀ alkyl, -OCF₃, -O(CH₂)₂SCH₃, -O(CH₂)₂-O-N(Rm)(Rn), and O-CH₂-C(=O)-N(Rm)(Rn), wherein each Rm and Rn is independently H or a substituted or unsubstituted C₁-C₁₀ alkyl. A "2'- modified sugar" means herein a furanosyl sugar modified at the 2' position.

A method for preparing a modified sugar is well known to those skilled in the art. In a nucleotide having a modified sugar moiety, the nucleobase moiety (natural one, modified one, or a combination thereof) may be maintained for hybridization with an appropriate nucleic acid target.

In general, modifications can be performed so that nucleotides in the same strand can independently undergo different modifications. In addition, to confer resistance to enzymatic cleavage, the same nucleotide may have a modified internucleoside bond (e.g., phosphorothioate bond), and also have a modified sugar (e.g., 2'-O-methyl modified sugar, or bicyclic sugar). Further, the same nucleotide can have a modified nucleobase (e.g., 5-methylcytosine), and also have a modified sugar (e.g., 2'-O-methyl modified sugar, or bicyclic sugar).

The number, type, and position of a non-natural nucleotide in a nucleic acid strand can influence the antisense effect or the like provided by a nucleic acid complex of the present invention. Selection of a modification may vary depending on the sequence of a target gene or the like, but one skilled in the art can determine a suitable embodiment by referring to descriptions in literatures related to the antisense method (for example, WO 2007/143315, WO 2008/043753, and WO 2008/049085). Furthermore, when the antisense effect of the nucleic acid complex after the modification is measured, if the measurement value thus obtained is not significantly lower than the measurement value for the nucleic acid complex before the modification (for example, in a case where the measurement value obtained after the modification is 70% or more, 80% or more, or 90% or more with respect to the measurement value for the nucleic acid complex before the modification), relevant modifications can be evaluated.

The term "complementary" as used herein means a relationship in which nucleobases can form so-called Watson-Crick base pairs (natural type base pairs), or non-Watson-Crick base pairs (Hoogsteen type base pairs, or the like) via hydrogen bonds. In the present invention, it is not necessarily required that a first nucleic acid strand is completely complementary to at least part of a target transcriptional product (e.g., the transcriptional product of a target gene), but it is permissible if the base sequence has a complementarity of at least 70%, preferably at least 80%, and more preferably at least 90% (e.g., 95%, 96%, 97%, 98%, or 99% or more). Similarly, it is not necessarily required that the complementary region in the second nucleic acid strand is completely complementary to at least part of the first nucleic acid strand, but it is permissible if the base sequence has a complementarity of at least 70%, preferably at least 80%, and more preferably at least 90% (e.g., 95%, 96%, 97%, 98%, or 99% or more).

An "alkyl group" herein means a linear or branched non-cyclic saturated aliphatic hydrocarbon. Examples of a C₁₋₃₅ linear or branched alkyl group include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, octyl, decyl, dodecyl, tridecyl, tetradecyl, 2,6,10-trimethylundecyl, pentadecyl, 3,7,11-trimethyldodecyl, hexadecyl, heptadecyl, 1-hexadecylheptadecyl, octadecyl, 6,10,14-trimethylpentadecan-2-yl, nonadecyl, 2,6,10,14-tetramethylpentadecyl, icosyl, 3,7,11,15-tetramethylhexadecyl, heneicosyl, docosyl, tricosyl, tetracosyl, pentacosyl, hexacosyl, heptacosyl, octacosyl, nonacosyl, triacontyl, hentriacontyl, dotriacontyl, tritriacontyl, tetratriacontyl, and pentatriacontyl.

The term "blood-brain barrier (BBB)" as used herein means a mechanism that selects and restricts substances which transition to the brain, and serves to protect the brain from harmful substances, as described above.

The term "blood-cerebrospinal fluid barrier (BCSFB)" as used herein means a mechanism present in the chorioid plexus that selects and restricts substances which transition to the brain as in the BBB mechanism.

The term "cerebrospinal fluid-brain barrier (CSFBB)" as used herein means a mechanism that selects and restricts substances which transition to the brain as in the BBB mechanism.

The term "central nervous system" as used herein means a tissue consisting of the brain and spinal cord, and constituting the nervous system together with the peripheral nervous system. The brain comprises cerebrum (cerebral cortex, cerebral white matter, and basal ganglion), diencephalon (thalamus, and subthalamic nucleus), cerebellum (cerebellar cortex, and cerebellar nucleus), and brainstem (midbrain, substantia nigra, pons, and medulla oblongata). Meanwhile, spinal cord comprises cervical spinal cord, thoracic spinal cord, lumbar spinal cord, sacral spinal cord, and coccygeal spinal cord. Although the central nervous system herein may be any of these regions, it may be preferably the cerebral cortex (frontal lobe, temporal lobe, parietal lobe, or occipital lobe), cerebellum, striatum, globus pallidus, claustrum, hippocampus, parahippocampal gyrus, brainstem, cervical spinal cord, thoracic spinal cord, or lumbar spinal cord.

The term "brain parenchyma" as used herein means a brain tissue consisting of a neuronal cell and/or a glial cell (e.g., astrocyte, oligodendrocyte, and microglia). The brain parenchyma comprises brain tissues of cerebrum, diencephalon, cerebellum, and brainstem (midbrain, hindbrain, and medulla oblongata).

A "salt thereof' means herein a salt of a nucleic acid complex of the present invention, and refers to a physiologically and pharmaceutically acceptable salt of a nucleic acid complex of the present invention, namely a salt that retains the desired biological activity of the nucleic acid complex, and does not have an undesired toxicological effect. Examples of such a salt include an alkali metal salt, such as a sodium salt, a potassium salt, and a lithium salt; an alkali earth metal salt, such as a calcium salt, and a magnesium salt; a metal salt, such as an aluminum salt, an iron salt, a zinc salt, a copper slat, a nickel salt, and a cobalt salt; an inorganic salt, such as an ammonium salt; an amine salt, such as a *t*-octylamine salt, a dibenzylamine salt, a morpholine salt, a glucosamine salt, a phenylglycine alkyl ester salt, a ethylenediamine salt, a guanidine salt, a diethylamine salt, a triethylamine salt, a dicyclohexylamine salt, a N,N'-dibenzylethylenediamine salt, a chloroprocaine salt, a procaine salt, a diethanolamine salt, a N-benzyl-phenethylamine salt, a piperazine salt, a tetramethylammonium salt, a tris(hydroxymethyl)aminomethane salt, a diolamine salt, and a meglumine salt; a hydrohalic acid salt, such as a hydrofluoric acid salt, a hydrochloric acid salt, a hydrobromic acid salt, and a hydroiodic acid salt; an inorganic acid salt, such as a nitrate, a perchlorate, a sulfate, and a phosphate; a lower alkane sulfonate, such as methanesulfonate, trifluoromethanesulfonate, and ethanesulfonate; an aryl sulfonate, such as benzene sulfonate, and p-toluene sulfonate; an organic salt, such as an acetate, a malate, a fumarate, a succinate, a citrate, a tartrate, an oxalate, and a maleate; and an amino acid salt, such as a glycine salt, a lysine salt, an arginine salt, an ornithine salt, a glutamate, and an aspartate.

In a certain embodiment, a nucleic acid complex of the present invention includes any pharmaceutically acceptable salt of the nucleic acid complex, an ester of the nucleic acid complex, or a salt of the ester. Examples of a suitable pharmaceutically acceptable salt include, but not limited to, a sodium salt, a potassium salt, and a meglumine salt.

### (Configuration of a first nucleic acid strand and a second nucleic acid strand)

In one aspect, the present invention relates to a nucleic acid complex comprising a first nucleic acid strand and a second nucleic acid strand.

The first nucleic acid strand is a single-stranded oligonucleotide strand that comprises a base sequence capable of hybridizing to at least part of a target transcriptional product, and provides an antisense effect on the target transcriptional product.

The second nucleic acid strand is a single-stranded oligonucleotide strand comprising a base sequence complementary to the first nucleic acid strand. The second nucleic acid strand is bound to a C₂₂₋₃₅ alkyl group optionally substituted with a hydroxy group, or an analog thereof. In a nucleic acid complex, the second nucleic acid strand is annealed to the first nucleic acid strand via hydrogen bonds of complementary base pairs.

In an embodiment, the present invention relates to a single-stranded nucleic acid strand bound to a C₂₂₋₃₅ alkyl group optionally substituted with a hydroxy group, or an analog thereof. The configuration of the single-stranded nucleic acid is the same as the first nucleic acid strand except that it is bound to a C₂₂₋₃₅ alkyl group optionally substituted with a hydroxy group, or an analog thereof. The configuration of the C₂₂₋₃₅ alkyl group optionally substituted with a hydroxy group, or an analog thereof, and the form of the linkage with a nucleic acid strand are as described herein in connection with a nucleic acid complex.

The base lengths of the first nucleic acid strand and the second nucleic acid strand may be usually, but not particularly limited to, at least 8 bases in length, at least 9 bases in length, at least 10 bases in length, at least 11 bases in length, at least 12 bases in length, at least 13 bases in length, at least 14 bases in length, or at least 15 bases in length. Further, the base lengths of the first nucleic acid strand and the second nucleic acid strand may be 35 bases in length or less, 30 bases in length or less, 25 bases in length or less, 24 bases in length or less, 23 bases in length or less, 22 bases in length or less, 21 bases in length or less, 20 bases in length or less, 19 bases in length or less, 18 bases in length or less, 17 bases in length or less, or 16 bases in length or less. The first nucleic acid strand and the second nucleic acid strand may be about 100 bases in length, or from 10 to 35 bases in length, from 12 to 25 bases in length, from 13 to 20 bases in length, from 14 to 19 bases in length, or from 15 to 18 bases in length. The first nucleic acid strand and the second nucleic acid strand may be in the same length or different lengths (e.g., either one may be shorter or longer by 1 to 3 bases). The double-stranded structure formed by the first nucleic acid strand and the second nucleic acid strand may comprise a bulge. The length may be selected especially according to the balance between the strength of the antisense effect and the specificity of the nucleic acid strand with respect to a target, among other factors such as, for example, cost, or synthesis yield.

The internucleoside bond in the first nucleic acid strand and the second nucleic acid strand may be a naturally occurring internucleoside bond and/or a modified internucleoside bond. Without limitation, at least one, at least two, or at least three internucleoside bonds from an end (5' end, 3' end or both the ends) of the first nucleic acid strand and/or the second nucleic acid strand are preferably modified internucleoside bonds. In this regard, for example, two internucleoside bonds from the end of a nucleic acid strand refers to an internucleoside bond closest to the end of the nucleic acid strand, and an internucleoside bond positioned next thereto on the opposite side to the end of the nucleic acid strand. Modified internucleoside bonds in the terminal region of a nucleic acid strand are preferred because they can reduce or inhibit undesired degradation of the nucleic acid strand. In an embodiment, all internucleoside bonds of the first nucleic acid strand and/or the second nucleic acid strand may be modified internucleoside bonds. The modified internucleoside bond may be a phosphorothioate bond.

In an embodiment, modified internucleoside bonds of the first nucleic acid strand and/or the second nucleic acid strand comprise non-negatively charged internucleoside bonds (which are each neutral or cationic) where the modified internucleoside bonds each exist as a neutral form or cationic form at a certain pH (e.g., a human physiological pH (about 7.4) or a pH at the delivered site (e.g., organelle, cell, tissue, organ, or organism)), rather than an anionic form (e.g., -O-P(O)(O⁻)-O- (an anionic form of a natural phosphate bond) or -O-P(O) (S⁻)-O- ((an anionic form of a phosphorothioate bond)). In an embodiment, the modified internucleoside bonds of the first nucleic acid strand and/or the second nucleic acid strand comprise neutral internucleoside bonds. In an embodiment, the modified internucleoside bonds of the first nucleic acid strand and/or the second nucleic acid strand comprise cationic internucleoside bonds. In an embodiment, the non-negatively charged internucleoside bond (e.g., neutral internucleoside bond), when is a neutral form, does not have any moiety having a pKa of less than 8, less than 9, less than 10, less than 11, less than 12, less than 13, or less than 14. In an embodiment, the non-negatively charged internucleoside bond is, for example, a methylphosphonate bond described in US Patent Nos. 5,264,423 and 5,286,717, a methylphosphotriester bond or an ethylphosphotriester bond described in US Patent No. 5,955,599, a methoxypropylphosphonate bond described in WO 2015/168172, or an internucleoside bond for use in self-neutralizing nucleic acid (ZON) described in WO 2016/081600. In an embodiment, the non-negatively charged internucleoside bond contains a triazole moiety or an alkyne moiety. In an embodiment, the non-negatively charged internucleoside bond contains a cyclic guanidine moiety. In an embodiment, the modified internucleoside bond containing a cyclic guanidine moiety has the following structure. 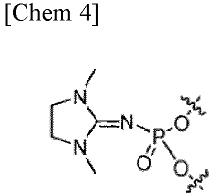 In an embodiment, a neutral internucleoside bond containing a cyclic guanidine moiety is chirally controlled. In an embodiment, the present disclosure relates to a composition comprising an oligonucleotide containing at least one neutral internucleoside bond and at least one phosphorothioate internucleoside bond. While not wishing to be bound by any particular theory, in at least some cases, a neutral internucleotide bond can improve properties and/or activity, for example, improve delivery, improve resistance to exonuclease and endonuclease, improve cellular uptake, improve endosomal escape, and/or improve nuclear uptake, as compared with an equivalent nucleic acid containing no neutral internucleotide bond.

In an embodiment, the first nucleic acid strand and the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50 or more modified internucleoside bonds. In an embodiment, the first nucleic acid strand and the second nucleic acid strand may each comprise at least 5%, at least 10%, at least 20%, at least 30%, at least 40%, at least 50%, at least 60%, at least 70%, at least 80%, at least 90% or more of modified internucleoside bonds.

In an embodiment, the first nucleic acid strand and the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50 or more internucleoside bonds chirally controlled. In an embodiment, the first nucleic acid strand and the second nucleic acid strand may each comprise at least 5%, at least 10%, at least 20%, at least 30%, at least 40%, at least 50%, at least 60%, at least 70%, at least 80%, at least 90% or more of internucleoside bonds chirally controlled.

In an embodiment, the first nucleic acid strand and the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50 or more non-negatively charged internucleoside bonds (preferably, neutral internucleoside bonds). In an embodiment, the first nucleic acid strand and the second nucleic acid strand may each comprise at least 5%, at least 10%, at least 20%, at least 30%, at least 40%, at least 50%, at least 60%, at least 70%, at least 80%, at least 90% or more of non-negatively charged internucleoside bonds.

At least one (e.g., three) internucleoside bond from the 3' end of the second nucleic acid strand may be a modified internucleoside bond, such as a phosphorothioate bond having high RNase resistance. It is preferable that an internucleoside bond such as a phosphorothioate modification is comprised at the 3' end of the second nucleic acid strand, because the gene inhibition activity of the double-stranded nucleic acid complex is enhanced.

At the 5' end and the 3' end of the second nucleic acid strand, internucleoside bonds for 2 to 6 bases at an end unbound to a C₂₂₋₃₅ alkyl group optionally substituted with a hydroxy group, or an analog thereof may be also modified internucleoside bonds (e.g., phosphorothioate bonds).

At least one (e.g., three) nucleoside from the 3' end of the second nucleic acid strand may be, for example, a modified nucleoside, such as 2'F-RNA, and 2'-OMe, which have high RNase resistance. It is preferable that a modified nucleoside such as 2'F-RNA, and 2'-OMe is comprised at the 3' end of the second nucleic acid strand, because the gene inhibition activity of the double-stranded nucleic acid complex is enhanced.

At the 5' end and the 3' end of the second nucleic acid strand, one to five nucleosides at the end unbound to a C₂₂₋₃₅ alkyl group optionally substituted with a hydroxy group, or an analog thereof may be, for example, modified nucleosides such as 2'F-RNA having high RNase resistance.

A nucleoside in the first nucleic acid strand and the second nucleic acid strand may be a natural nucleoside (deoxyribonucleoside, ribonucleoside, or both) and/or a non-natural nucleoside.

In an embodiment, the first nucleic acid strand and the second nucleic acid strand each comprise a cationic nucleoside.

Since the base sequence of the first nucleic acid strand is herein complementary to at least part of the base sequence of a target transcriptional product, it can hybridize (or anneal) to the target transcriptional product. The complementarity of a base sequence can be determined using a BLAST program or the like. One skilled in the art can readily determine the conditions (temperature, salt concentration, etc.) under which the two strands can be hybridized, taking into account the complementarity between the strands. Further, one skilled in the art can readily design an antisense nucleic acid that is complementary to the target transcriptional product based on, for example, information on the base sequence of a target gene.

Hybridization conditions may be a variety of stringent conditions, such as a low-stringent condition and a high-stringent condition. A low-stringent condition may be a condition with a relatively low temperature, and a high salt concentration, for example, 30°C, 2 × SSC, and 0.1% SDS. A high-stringent condition may be a condition with a relatively high temperature, and a low salt concentration, for example, 65°C, 0.1 × SSC, and 0.1% SDS. The stringency of hybridization can be adjusted by varying the conditions, such as temperature and salt concentration. Here, 1 × SSC contains 150 mM sodium chloride and 15 mM sodium citrate.

The first nucleic acid strand may comprise at least four, at least five, at least six, or at least seven contiguous nucleosides that are recognized by RNase H when hybridized to a target transcriptional product. Typically, it can be a region comprising contiguous nucleosides of from 4 to 20 bases, from 5 to 16 bases, or from 6 to 12 bases. As a nucleoside that can be recognized by RNase H, for example, a natural deoxyribonucleoside can be used. A suitable nucleoside comprising a modified deoxyribonucleoside and other bases are well known in the art. It is also known that a nucleoside having a hydroxy group at the 2' position, such as a ribonucleoside, is unsuitable as said nucleoside. The suitability of a nucleoside for use in the region comprising "at least four contiguous nucleosides" can be readily determined. In an embodiment, the first nucleic acid strand may comprise at least four contiguous deoxyribonucleosides.

In an embodiment, the full length of the first nucleic acid strand and/or the second nucleic acid strand is not constituted solely by natural ribonucleosides. In the first nucleic acid strand and/or the second nucleic acid strand, natural ribonucleosides should preferably be less than half of the full length, or should not be contained.

In an embodiment, the second nucleic acid strand may comprise at least four contiguous ribonucleosides complementary to the above-described at least four contiguous nucleosides (e.g., deoxyribonucleosides) in the first nucleic acid strand. This is for the purpose that the second nucleic acid strand forms a partial DNA-RNA heteroduplex with the first nucleic acid strand, which can then be recognized and cleaved by RNase H. The at least four contiguous ribonucleosides in the second nucleic acid strand are preferably linked by naturally occurring internucleoside bonds, namely phosphodiester bonds.

In the second nucleic acid strand, all the nucleosides may be composed of ribonucleosides and/or modified nucleosides. All the nucleosides in the second nucleic acid strand may be composed of deoxyribonucleosides and/or modified nucleosides, or the second nucleic acid strand may not comprise a ribonucleoside.

The first nucleic acid strand and/or the second nucleic acid strand constituting a nucleic acid complex of the present invention may be a gapmer. A "gapmer" means herein a single-stranded nucleic acid consisting of a central region (DNA gap region), and a 5' wing region and a 3' wing region located respectively on the 5' end side and the 3' end side of the central region. The central region in a gapmer comprises at least four contiguous deoxyribonucleosides, and the 5' wing region and the 3' wing region comprise a non-natural nucleoside. When the non-natural nucleoside constituting a 5' wing region and a 3' wing region comprises a bridged nucleoside, or consists thereof, the gapmer is specifically referred to as "BNA/DNA gapmer". When the non-natural nucleoside constituting a 5' wing region and a 3' wing region comprises a peptide nucleic acid, or consists thereof, the gapmer is specifically referred to as "peptide nucleic acid gapmer". When the non-natural nucleoside constituting a 5' wing region and a 3' wing region comprises a peptide nucleic acid, or consists thereof, the gapmer is specifically referred to as "morpholino nucleic acid gapmer". The number of bridged nucleoside comprised in the 5' wing region and the 3' wing region may be two or three. The bridged nucleoside comprised in the 5' wing region and the 3' wing region may exist contiguously or noncontiguously in the 5' wing region and the 3' wing region. A bridged nucleoside may further comprise a modified nucleobase (e.g., 5-methylcytosine). When the bridged nucleoside is an LNA nucleoside, the gapmer is referred to as "LNA/DNA gapmer". Each of the base length of the 5' wing region and the 3' wing region may be independently at least 2 bases in length, e.g., from 2 to 10 bases in length, from 2 to 7 bases in length, or from 3 to 5 bases in length. The 5' wing region and the 3' wing region may comprise at least one kind of non-natural nucleoside, and may further comprise a natural nucleoside.

The first nucleic acid strand and/or the second nucleic acid strand constituting the gapmer may be composed of bridged nucleosides from 2 to 7 bases in length or from 3 to 5 bases in length, ribonucleosides or deoxyribonucleosides from 4 to 15 bases in length, or from 8 to 12 bases in length, and bridged nucleosides from 2 to 7 bases in length, or from 3 to 5 bases in length from the 5' end in this order.

The first nucleic acid strand and/or the second nucleic acid strand constituting a nucleic acid complex of the present invention may be a mixmer. A "mixmer" means herein a nucleic acid strand which alternatingly comprises natural nucleosides and non-natural nucleosides having periodic, or random segment lengths, and does not comprise four or more contiguous deoxyribonucleosides, or ribonucleosides. Among mixmers, a mixmer in which the non-natural nucleoside is a bridged nucleoside, and the natural nucleoside is a deoxyribonucleoside, is specifically called "BNA/DNA mixmer". Among mixmers, a mixmer in which the non-natural nucleoside is a peptide nucleic acid, and the natural nucleoside is a deoxyribonucleoside, is specifically called "peptide nucleic acid/DNA mixmer". Among mixmers, a mixmer in which the non-natural nucleoside is a morpholino nucleic acid, and the natural nucleoside is a deoxyribonucleoside, is specifically called "morpholino nucleic acid/DNA mixmer". A mixmer is not limited to comprise only two kinds of nucleosides. A mixmer may comprise any number of kinds of nucleosides, irrespective of a natural or modified nucleoside, or a nucleoside mimic. For example, it may comprise one or two contiguous deoxyribonucleosides separated by a bridged nucleoside (e.g., LNA nucleoside). A bridged nucleoside may further comprise a modified nucleobase (e.g., 5-methylcytosine).

At least one, at least two, at least three, or at least four nucleosides from an end (5' end, 3' end, or both the ends) of the second nucleic acid strand may be modified nucleosides. The modified nucleoside may comprise a modified sugar and/or a modified nucleobase. The modified sugar may be a 2'-modified sugar (e.g., a sugar comprising a 2'-O-methyl group). The modified nucleobase may be 5-methylcytosine.

The second nucleic acid strand may be composed of modified nucleosides (e.g., modified nucleosides comprising a 2'-modified sugar) from 2 to 7 bases in length, or from 3 to 5 bases in length, ribonucleosides or deoxyribonucleosides from 4 to 15 bases in length, or from 8 to 12 bases in length (optionally linked by a modified internucleoside bond), and modified nucleosides (e.g., a modified nucleoside comprising a 2'-modified sugar) from 2 to 7 bases in length or from 3 to 5 bases in length from the 5' end in this order. In this case, the first nucleic acid strand may be a gapmer.

The first nucleic acid strand and the second nucleic acid strand, as a whole or in part, may comprise a nucleoside mimic or a nucleotide mimic. A nucleotide mimic may be a peptide nucleic acid and/or a morpholino nucleic acid. The first nucleic acid strand may comprise at least one modified nucleoside. The modified nucleoside may comprise a 2'-modified sugar. The 2'-modified sugar may be a sugar comprising a 2'-O-methyl group.

The first nucleic acid strand and the second nucleic acid strand, as a whole or in part, may comprise a cationic nucleoside. In an embodiment, a cationic nucleoside is a nucleoside comprising amine bridged nucleic acid (also known as 2'-Amino-LNA) (e.g., 3-(Bis(3-aminopropyl)amino)propanoyl substitute), aminoalkyl-modified nucleic acid (e.g., 2'-O-methyl and 4'-CH₂CH₂CH₂NH₂ substitute), GuNA (e.g., in Figure 3, R=H is GuNA[N-H] and R=Me is GuNA[N-Me]), or the like.

The first nucleic acid strand and the second nucleic acid strand may comprise any combination of the modified internucleoside bond and the modified nucleoside described above.

The second nucleic acid strand is bound to a C₂₂₋₃₅ alkyl group optionally substituted with a hydroxy group, or an analog thereof. Those skilled in the art can produce a C₂₂₋₃₅ alkyl group optionally substituted with a hydroxy group, or an analog thereof, using a publicly know method.

An "analog" herein refers to a compound having a similar structure and property, having the same or a similar basic backbone. An analog comprises, for example, a biosynthetic intermediate, and a metabolite. Those skilled in the art can determine whether a compound is an analog of another compound.

In an embodiment, a C₂₂₋₃₅ alkyl group optionally substituted with a hydroxy group (e.g., docosyl, tricosyl, tetracosyl, pentacosyl, hexacosyl, heptacosyl, octacosyl, nonacosyl, triacontyl, or 1-hexadecylheptadecyl) is, for example, a C₂₂₋₂₆ or C₂₈ alkyl group optionally substituted with a hydroxy group (e.g., docosyl, tricosyl, tetracosyl, pentacosyl, hexacosyl, or octacosyl), for example, a C₂₂ alkyl group optionally substituted with a hydroxy group (e.g., docosyl). In an embodiment, a C₂₂₋₃₅ alkyl group optionally substituted with a hydroxy group is an unsubstituted C₂₂₋₂₆ or C₂₈ alkyl group (e.g., docosyl, tricosyl, tetracosyl, pentacosyl, hexacosyl, or octacosyl), for example, an unsubstituted C₂₂₋₂₄ or C₂₆ alkyl group (e.g., docosyl, tricosyl, tetracosyl, or hexacosyl), for example, an unsubstituted C₂₂ alkyl group (e.g., docosyl). An alkyl group may be any of a linear alkyl group and a branched alkyl group. In an embodiment, a C₂₂₋₃₅ alkyl group optionally substituted with a hydroxy group is an unsubstituted linear C₂₂₋₂₆ or linear C₂₈ alkyl group (e.g., docosyl, tricosyl, tetracosyl, pentacosyl, hexacosyl, or octacosyl), for example, an unsubstituted linear C₂₂₋₂₄ or linear C₂₆ alkyl group (e.g., docosyl, tricosyl, tetracosyl, or hexacosyl), for example, an unsubstituted linear C₂₂ alkyl group (e.g., docosyl). In an embodiment, a C₂₂₋₃₅ alkyl group optionally substituted with a hydroxy group is an unsubstituted branched C₃₃ alkyl group (e.g., 1-hexadecylheptadecyl).

A C₂₂₋₃₅ alkyl group optionally substituted with a hydroxy group or an analog thereof may be linked to the 5' end, the 3' end, or both the ends of the second nucleic acid strand. Alternatively, a C₂₂₋₃₅ alkyl group optionally substituted with a hydroxy group or the analog thereof may be linked to an interior nucleotide in the second nucleic acid strand. For example, a C₂₂₋₃₅ alkyl group optionally substituted with a hydroxy group or an analog thereof can be bound to the 5' end of the second nucleic acid strand. In other embodiments, the second nucleic acid strand may comprise two or more of a C₂₂₋₃₅ alkyl group optionally substituted with a hydroxy group or an analog thereof, which may be bound to the second nucleic acid strand at a plurality of positions, and/or bound to the second nucleic acid strand as a group at a single position. A C₂₂₋₃₅ alkyl group optionally substituted with a hydroxy group or an analog thereof may be linked one each to the 5' end and the 3' end of the second nucleic acid strand. A C₂₂₋₃₅ alkyl group optionally substituted with a hydroxy group or an analog thereof may be linked one each to the 5' end and the 3' end of the second nucleic acid strand.

The binding between the second nucleic acid strand and a C₂₂₋₃₅ alkyl group optionally substituted with a hydroxy group or an analog thereof may be either a direct binding or an indirect binding. A direct binding means that two molecules are directly bound. The indirect binding means that the two molecules to be bound are bound via another substance.

In an embodiment, the binding between the second nucleic acid strand and a C₂₂₋₃₅ alkyl group optionally substituted with a hydroxy group or an analog thereof may be to a nucleotide at the 5' end, the 3' end of the second nucleic acid strand or interiorly in the second nucleic acid strand via a phosphate ester bond or a phosphorothioate bond.

In an embodiment, the binding is to the 5' end of the second nucleic acid strand via a phosphate ester bond or a phosphorothioate bond.

In an embodiment, the binding is to the 5' end of the second nucleic acid strand via a phosphate ester bond.

When the second nucleic acid strand and a C₂₂₋₃₅ alkyl group optionally substituted with a hydroxy group or an analog thereof are indirectly bound, they may be bound via a linking group (herein frequently referred to as a "linker"). A linker may be bound to a nucleotide at the 5' end, the 3' end of the second nucleic acid strand or interiorly in the second nucleic acid strand via a phosphate ester bond or a phosphorothioate bond.

In an embodiment, the linker is bound to the 5' end of the second nucleic acid strand via a phosphate ester bond or a phosphorothioate bond.

In an embodiment, a linker is bound to the 5' end of the second nucleic acid strand via a phosphate ester bond.

When the second nucleic acid strand and a C₂₂₋₃₅ alkyl group optionally substituted with a hydroxy group or an analog thereof are indirectly bound, they may be bound via a cleavable linker. A "cleavable linker" refers to a linking group that can be cleaved under physiological conditions, for example, in a cell or in an animal body (e.g., in a human body). A cleavable linker may be selectively cleaved by an endogenous enzyme, such as a nuclease, and a peptidase, under acidic conditions, in a reductive environment, etc. Specific examples thereof include an amide bond, an ester bond, a phosphate ester bond, either or both ester bonds of a phosphodiester bond, a carbamate bond, and a disulfide bond, as well as a nucleotide linker such as a natural DNA linker.

When the second nucleic acid strand and a C₂₂₋₃₅ alkyl group optionally substituted with a hydroxy group or an analog thereof are indirectly bound, they may also be bound via an uncleavable linker. An "uncleavable linker" refers to a linking group that is not cleavable under physiological conditions. Examples of such an uncleavable linker include a phosphorothioate bond, and a linker consisting of modified or unmodified deoxyribonucleosides, or modified or unmodified ribonucleosides linked by a phosphorothioate bond.

Although there is no particular restriction on the strand length of a cleavable linker, or an uncleavable linker in the case of a nucleic acid such as DNA, or an oligonucleotide, it is usually from 1 to 20 bases in length, from 1 to 10 bases in length, or from 1 to 6 bases in length.

As a specific example of said linker, there is a linker represented by the following Formula (II): 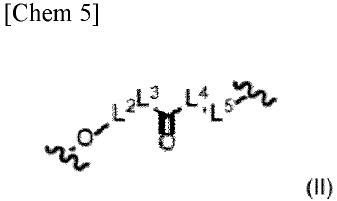 (wherein L² represents a substituted or unsubstituted C₁₋₁₂ alkylene group (e.g., propylene, hexylene, or dodecylene), a substituted or unsubstituted C₃₋₈ cycloalkylene group (e.g., cyclohexylene), -(CH₂)₂-O-(CH₂)₂-O-(CH₂)₂-O-(CH₂)₃-, or CH(CH₂-OH)-CH₂-O-(CH₂)₂-O-(CH₂)₂-O-(CH₂)₂-O-(CH₂)₃-; L³ represents -NH- or a bond; and L⁴ represents a substituted or unsubstituted C₁₋₁₂ alkylene group (e.g., ethylene, pentylene, heptylene, or undecylene), a substituted or unsubstituted C₃₋₈ cycloalkylene group (e.g., cyclohexylene), -(CH₂)₂-[O-(CH₂)₂]ₘ-, or a bond, wherein m represents an integer of 1 to 25; and L⁵ represents -NH-(C=O)-, -(C=O)-, or a bond (wherein the substitution is preferably done with a halogen atom).

In an embodiment, the linker represented by Formula (II) is a linker in which L² is an unsubstituted C₃-C₆ alkylene group (e.g., propylene, hexylene), -(CH₂)₂-O-(CH₂)₂-O-(CH₂)₂-O-(CH₂)₃-, or -(CH₂)₂-O-(CH₂)₂-O-(CH₂)₂-O-(CH₂)₂-O-(CH₂)₃-, L³ is -NH-, and L⁴ and L⁵ are a bond.

As a specific example of said linker, there is a linker represented by the following Formula (I):

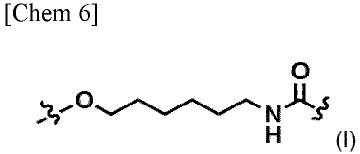

In a certain embodiment, the linker comprises one or more groups selected from an alkyl group, an amino group, an oxo group, an amide group, a disulfide group, a polyethylene glycol group, an ether group, a thioether group, and a hydroxylamino group. In such a certain embodiment, the linker comprises a group selected from an alkyl group, amino group, an oxo group, an amide group, and an ether group. In a certain embodiment, the linker comprises a group selected from an alkyl group and an amide group. In a certain embodiment, the linker comprises a group selected from an alkyl group and an ether group. In a certain embodiment, the linker comprises at least one phosphorus moiety. In a certain embodiment, the linker comprises at least one phosphate group. In a certain embodiment, the linker comprises at least one neutral conjugate group.

In a certain embodiment, the linker comprises a bifunctional binding moiety. In general, a bifunctional binding moiety comprises at least two functional groups. One of the functional groups is selected so as to be bound to a specified site on the second nucleic acid strand, and the other one is selected so as to be bound to a C₂₂₋₃₅ alkyl group optionally substituted with a hydroxy group or an analog thereof. Examples of such a functional group for use in the bifunctional binding moiety include, but not limited to, an electrophile reactive with a nucleophilic group and a nucleophile reactive with an electrophilic group. In a certain embodiment, the bifunctional binding moiety comprises one or more groups selected from amino, hydroxyl, carboxylic acid, thiol, alkyl, alkenyl, and alkynyl.

Examples of the linker include, but not limited to, pyrrolidine, 8-amino-3,6-dioxaoctanoic acid (ADO), succinimidyl 4-(N-maleimidemethyl)cyclohexane-1-carboxylate (SMCC), and 6-aminohexanoic acid (AHEX or AHA). Other linkers include, but not limited to, optionally substituted C₁₋₁₀ alkyl, optionally substituted C₂₋₁₀ alkenyl, or optionally substituted C₂₋₁₀ alkynyl, and non-limiting lists of a preferable substituent include hydroxyl, amino, alkoxy, carboxy, benzyl, phenyl, nitro, thiol, thioalkoxy, halogen, alkyl, aryl, alkenyl, and alkynyl.

In a certain embodiment, the linker comprises 1 to 10 linker nucleosides. In a certain embodiment, a conjugate linker comprises 2 to 5 linker nucleosides. In a certain embodiment, the linker precisely comprises 3 linker nucleosides. In a certain embodiment, the linker comprises a TCA motif. In a certain embodiment, the linker nucleoside is a non-natural nucleoside. In a certain embodiment, the linker nucleoside comprises a modified sugar moiety. In a certain embodiment, the linker nucleoside is a natural nucleoside. In a certain embodiment, the linker nucleoside comprises a heterocyclic base optionally protected, selected from a purine base, a modified purine base, a pyrimidine base, or a modified pyrimidine base. A linker nucleoside is herein not considered to be a part of oligonucleotide. Therefore, in an embodiment where an oligomeric compound comprises an oligonucleotide consisting of nucleosides of a specified number or in a specified range and/or having a specified percent complementarity relative to a reference nucleic acid and the oligomeric compound comprises a linker comprising a linker nucleoside, the linker nucleoside is not used in calculation of the length of an oligonucleotide and is not used in determination of the percent complementarity of an oligonucleotide relative to a reference nucleic acid.

In a certain embodiment, in a case where a nucleic acid complex of the present invention comprises optical isomers, stereoisomers, regioisomers, or rotational isomers, these may also be comprised as a nucleic acid complex of the present invention, and each may be obtained as an isolated product by a publicly known synthesis method and a separation method. For example, if optical isomers are present in a nucleic acid complex of the present invention, the optical isomers separated from the compound are also encompassed in the nucleic acid complex of the present invention.

In a certain embodiment, a nucleic acid complex of the present invention comprises a prodrug and a pharmaceutically acceptable salt of the prodrug. A prodrug of a nucleic acid complex of the present invention and a pharmaceutically acceptable salt of the prodrug refer to compounds that are converted to a nucleic acid complex of the present invention by a reaction with an enzyme, gastric acid, or the like under physiological conditions in vivo, namely a compound that are changed into a nucleic acid complex of the present invention by enzymatically causing oxidation, reduction, or hydrolysis, or a compound that are changed into a nucleic acid complex of the present invention by hydrolysis caused by gastric acid, or the like. In a certain embodiment, a prodrug of a nucleic acid complex comprises one or more of a C₂₂₋₃₅ alkyl group optionally substituted with a hydroxy group, or an analog thereof bound to the first nucleic acid strand or the second nucleic acid strand.

With respect to a nucleic acid complex of the present invention, an antisense effect of the first nucleic acid strand on a target transcriptional product can be measured by a method publicly known in the field. For example, it can be measured by using a publicly known technique, such as northern blotting, quantitative PCR, or western blotting, after introducing a nucleic acid complex into cells or the like. Specifically, it may be performed by examining a decrease in the expression amount of a target gene or the level of a target transcriptional product in cells (e.g., the amount of RNA such as the amount of mRNA or the amount of microRNA, the amount of cDNA, or the amount of a protein) by an antisense effect, using the aforedescribed publicly known technique.

Measurement of the antisense effect of a nucleic acid complex of the present invention in the central nervous system, and judgement of crossing BBB and so on can also be performed by a method publicly known in the art. For example, but without limitation, such judgment is possible by measuring whether the expression amount of a target gene or the level of a target transcriptional product in the central nervous system is inhibited or not after several days to several months (e.g., after 2 to 7 days, or 1 month) from administration of a nucleic acid complex of the present invention to a subject (e.g., mouse). As for the judgment criteria, in a case where a measurement value for the expression amount of a target gene or the level of a target transcriptional product is decreased by at least 5%, at least 10%, at least 15%, at least 20%, at least 25%, at least 30%, or at least 40% compared with a measurement value for a negative control (e.g., vehicle administration), it may be judged that the nucleic acid complex of the present invention has crossed BBB and so on, and produced an antisense effect in the central nervous system. Further, the judgement on crossing BBB and so on can also be made by measuring the abundance (concentration) of a nucleic acid complex of the present invention in the central nervous system after several days to several months (e.g., after 2 to 7 days, or 1 month) from administration of a nucleic acid complex of the present invention to a subject (e.g., mouse).

Exemplary embodiments of a nucleic acid complex of the present invention are described above. However, a nucleic acid complex of the present invention is not limited to the above exemplary embodiments. Further, one skilled in the art can produce a first nucleic acid strand and a second nucleic acid strand constituting various embodiments of a nucleic acid complex of the present invention by appropriately selecting a publicly known method. For example, a nucleic acid molecule of the present invention can be produced by designing each nucleic acid molecule based on the information on the base sequence of a target transcriptional product (e.g., the base sequence of a target gene), synthesizing the nucleic acid using a commercially available automatic nucleic acid synthesizer, such as the products of GE Healthcare, Thermo Fisher Scientific, and Beckman Coulter, and then purifying the obtained oligonucleotides using, e.g., a reversed phase column.

The second nucleic acid strand may further comprise at least one functional moiety bound to the polynucleotide. There is no particular restriction on the structure of a "functional moiety" in a specific embodiment insofar as the functional moiety imparts a desired function to a nucleic acid complex and/or the strand to which the functional moiety is bound. Examples of the desired function include a labeling function, and a purifying function. Examples of a moiety that provides a labeling function include compounds such as a fluorescent protein, and luciferase. Examples of a moiety that provides a purifying function include compounds such as biotin, avidin, His-tag peptide, GST-tag peptide, and FLAG-tag peptide. A molecule having an activity for delivery of a double-stranded nucleic acid complex in an embodiment to a target site is preferably bound as a functional moiety to the second nucleic acid strand, from the viewpoint that the first nucleic acid strand is efficiently delivered to a target site at a high specificity and expression of a target gene is very effectively suppressed by the nucleic acid. Examples of a moiety for providing a delivery function to a target include lipid, antibody, aptamer, and a ligand to a particular receptor. The binding position and the type of binding of the functional moiety in the second nucleic acid strand are as described above in connection with the binding of a C₂₂₋₃₅ alkyl group optionally substituted with a hydroxy group or an analog thereof to the second nucleic acid strand.

In an embodiment, the second nucleic acid strand is bound to a lipid. Examples of a lipid include, but not limited to, tocopherol, cholesterol, fatty acid, phospholipid, and analogs thereof; folic acid, vitamin C, vitamin B1, vitamin B2; estradiol, androstane, and analogs thereof; steroid and an analog thereof; ligand of LDLR, SRBI or LRP1/2; FK-506, and cyclosporine.

In an embodiment, a nucleic acid complex to which a functional moiety is bound can be produced by using a nucleic acid species to which a functional moiety has been bound in advance, and performing the synthesis, purification, and annealing as described above. For example, the second nucleic acid strand can be produced by performing the above synthesis and purification using a nucleic acid species to which a C₂₂₋₃₅ alkyl group optionally substituted with a hydroxy group or an analog thereof has been bound in advance.

In an embodiment, a C₂₂₋₃₅ alkyl group optionally substituted with a hydroxy group, or an analog thereof can be bound by a publicly known method to a second nucleic acid strand produced by performing the above synthesis and purification. The method for linking a functional moiety to a nucleic acid is well known in the art. Nucleic acids produced by this method are mixed in an appropriate buffer solution, denatured in a range of about 90°C to 98°C for several minutes (e.g., for 5 min), and then the nucleic acids are annealed in a range of about 30°C to 70°C for about 1 to 8 hours to prepare one nucleic acid complex of the present invention. Alternatively, a nucleic acid strand is available by ordering from various manufacturers (e.g., GeneDesign Inc.), by specifying the base sequence, and the modified position and type. The above annealing step can be performed by leaving the solution at room temperature (about 10°C to about 35°C) for about 5 to 60 min.

The first nucleic acid strand and the second nucleic acid strand are each dissolved in a buffer solution (e.g., phosphate-buffered saline) or in water in a range of about 70°C to 98°C, and the obtained two solutions are mixed. The mixed liquid may be kept in a range of about 70°C to 98°C for several minutes (e.g., 5 min), and then in a range of about 30°C to 70°C (or 30°C to 50°C) for about 1 to 8 hours to prepare a nucleic acid complex of some embodiments of the present invention. The first nucleic acid strand and the second nucleic acid strand may be each dissolved in a buffer solution (e.g., phosphate-buffered saline) or water at room temperature (about 10°C to about 35°C).

However, the conditions (time and temperature) for annealing at the time of preparing a nucleic acid complex are not limited to the aforedescribed conditions. Conditions suitable for promoting the annealing of a nucleic acid strand are well known in the art.

### (Effect of nucleic acid complex)

The nucleic acid complex of the present invention can inhibit the effect of a target miRNA in the central nervous system of a subject. Specific examples include a nucleic acid complex, in which a first nucleic acid strand comprises a base sequence capable of hybridizing to at least part of a target miRNA and has an antisense effect on the target miRNA; a second nucleic acid strand comprises a base sequence complementary to the first nucleic acid strand, and is bound to a C₂₂₋₃₅ alkyl group optionally substituted with a hydroxy group or an analog thereof; and the first nucleic acid strand and the second nucleic acid strand are annealed each other. By inhibiting the effect of the target miRNA by this nucleic acid complex, the expression of a gene that is normally downregulated by the target miRNA can be upregulated.

The nucleic acid complex of the present invention can regulate expression or editing of a target RNA in the central nervous system of a subject. Specific examples include a nucleic acid complex, in which a first nucleic acid strand comprises a base sequence capable of hybridizing to at least part of a target RNA, and has an antisense effect on the target RNA; and a second nucleic acid strand comprises a base sequence complementary to the first nucleic acid strand, and is bound to a C₂₂₋₃₅ alkyl group optionally substituted with a hydroxy group or an analog thereof; and the first nucleic acid strand and the second nucleic acid strand are annealed each other. Here, "expression regulation of a target RNA" includes, for example, up-regulation and down-regulation of the expression amount. Further, "regulation of editing of a target RNA" includes regulation of splicing by RNA editing, e.g., exon skipping and exon inclusion. The target RNA may be a viral or bacterial RNA, or a toxic RNA.

The nucleic acid complex of the present invention can inhibit the translation of a target mRNA in the central nervous system of a subject. Specific examples include a nucleic acid complex, in which a first nucleic acid strand comprises a base sequence capable of hybridizing to at least part of a target mRNA, and has an antisense effect on the target mRNA; and a second nucleic acid strand comprises a base sequence complementary to the first nucleic acid strand, and is bound to a C₂₂₋₃₅ alkyl group optionally substituted with a hydroxy group or an analog thereof; and the first nucleic acid strand and the second nucleic acid are annealed each other. This nucleic acid complex causes a steric block to inhibit the translation of mRNA by binding of the first nucleic acid strand to the target mRNA.

The nucleic acid complex of the present invention can be effectively used, without limitation, in the regulation of expression or editing of a target transcriptional product in the microglia in the central nervous system.

### <Composition>

The second aspect of the present invention is a composition. The composition of the present invention comprises the nucleic acid complex of the first aspect, or a single-stranded nucleic acid strand as an active ingredient and/or as a drug delivery molecule. The nucleic acid complex of the first aspect, or a single-stranded nucleic acid strand can cross BBB and so on and regulate the expression amount of a target transcriptional product in the central nervous system by an antisense effect (e.g., the expression amount is decreased). In an embodiment, the nucleic acid complex of the first aspect, or a single-stranded nucleic acid strand can transition from blood to the brain (brain parenchyma) and regulate the expression amount of a target transcriptional product in the brain parenchyma by an antisense effect (e.g., the expression amount is decreased). Therefore, the composition of the present invention may be a composition or a pharmaceutical composition that delivers a nucleic acid complex for treating a subject by administering the same to the subject. In an embodiment, the composition of the present invention is a composition for regulating the expression or editing of a target transcriptional product in the central nervous system, and/or for delivering a drug to the central nervous system. The composition of the present invention may be effectively used, without limitation, in regulating the expression or editing of a target transcriptional product in the microglia in the central nervous system.

Further, an embodiment of the present invention relates to a therapeutic method for treating each central nervous system disease by administering a composition comprising a nucleic acid complex.

### (Formulation)

The composition herein can be formulated by a publicly known method. For example, the present composition can be used perorally or parenterally in a form of capsule, tablet, pill, liquid formulation, dispersant, granule, microgranule, film-coated tablet, pellet, lozenge, sublingual formulation, peptizer, buccal tablet, paste, syrup, suspension, elixir, emulsion, coating formulation, ointment, plaster, cataplasm, transdermal patch, lotion, inhalant, aerosol, eye drop, injection, and suppository.

In formulating these formulations, a pharmaceutically acceptable carrier or solvent, or a carrier or solvent acceptable as a food and beverage may be incorporated as appropriate. Specific examples of such a carrier or solvent include sterile water, physiological saline, vegetable oil, base, emulsifier, suspending agent, surfactant, pH adjuster, stabilizer, flavor, perfume, excipient, vehicle, preservative, binder, diluent, isotonizing agent, sedatives, bulking agent, disintegrating agent, buffer, coating agent, lubricant, colorant, sweetening agent, thickener, flavoring substance, dissolution aid, and other additives.

### (Administration mode, dosage)

There is no particular restriction herein on a preferable administration mode of the composition. For example, it may be peroral administration or parenteral administration. Specific examples of parenteral administration include intravenous administration, intraarterial administration, intraperitoneal administration, subcutaneous administration, intradermal administration, tracheal/bronchial administration, rectal administration, intrathecal administration, intracerebroventricular administration, nasal administration, and intramuscular administration, as well as administration by blood transfusion. Administration by intramuscular injection, intravenous infusion, or implanted continuous subcutaneous administration is also possible. Since self-injection by a patient is possible in the case of subcutaneous administration, it is suitable. In the case of intravenous administration, the amount of a nucleic acid complex contained in a single dose of the composition, namely the single dose of a nucleic acid complex may be, for example, 0.001 mg/kg or more, 0.005 mg/kg or more, 0.01 mg/kg or more, 0.025 mg/kg or more, 0.1 mg/kg or more, 0.5 mg/kg or more, 1 mg/kg or more, 2.5 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any amount within the range of 0.001 mg/kg to 500 mg/kg (e.g., 0.001 mg/kg, 0.01 mg/kg, 0.1 mg/kg, 1 mg/kg, 5 mg/kg, 10 mg/kg, 50 mg/kg, 100 mg/kg, or 200 mg/kg) can be selected as appropriate.

### (Subject, application subject)

A "subject" herein refers to a subject to which the composition of the present invention is applied. The subject includes an individual as well as an organ, a tissue, and a cell. In a case where the subject is an individual, the composition of the present invention may be applied to any animals, including humans. Subjects other than humans may include, for example, various domestic animals, poultry, pets, and laboratory animals. The subject may be an individual that needs reduction in the expression amount of a target transcriptional product in the central nervous system, or an individual that needs a treatment for a central nervous system disease.

The composition of the present invention can reduce the expression amount of the target transcriptional product in the central nervous system due to a transitioning effect from blood to the brain (e.g., an effect of crossing BBB and so on) and an antisense effect of the nucleic acid complex of the first aspect, or a single-stranded nucleic acid strand that are contained therein.

When the composition of the present invention is applied for treating a central nervous system disease, the disease to be treated is preferably a central nervous system disease related to increase or decrease in the gene expression, especially a disease related to increase in a target transcriptional product, or the expression of a target gene (e.g., tumor). Examples thereof include, but not limited to, brain tumor, Alzheimer's disease, Parkinson's disease, amyotrophic lateral sclerosis, multiple sclerosis, and Huntington's disease.

In an embodiment, the composition of the present invention is applied for treating an immune-mediated central nervous system disease. Examples of an immune-mediated central nervous system disease include a microglia-associated disease, and examples of a microglia-associated disease include Alzheimer's disease, multiple sclerosis, ALS, and neuropathic pains.

Although there is no particular restriction on the delivery site of a composition of the present invention, more specifically the delivery site of an active ingredient contained in the composition, when it is delivered to an appropriate site depending on each disease, more effective results can be obtained. To give a specific example, in the treatment of Alzheimer's disease, drug delivery to hippocampus and/or the parietal lobe may be effective. Further, in the treatment of frontotemporal dementia (FTD) (including frontotemporal lobar degeneration (FTLD), semantic dementia (SD), and progressive nonfluent aphasia (PNFA)), or Pick's disease, drug delivery to frontal lobe, temporal lobe and/or substantia nigra may be effective. Further, in the treatment of Parkinson's disease dementia, drug delivery to occipital lobe, substantia nigra and/or striatum may be effective. In the treatment of Parkinson's disease, drug delivery to substantia nigra and/or striatum may be effective. In the treatment of corticobasal degeneration (CBD), drug delivery to frontal lobe, parietal lobe, basal ganglion, and/or substantia nigra may be effective. In the treatment of progressive supranuclear paralysis (PSP), drug delivery to frontal lobe, basal ganglion and/or substantia nigra may be effective. In the treatment of amyotrophic lateral sclerosis, drug delivery to frontal lobe, parietal lobe, basal ganglion and/or substantia nigra may be effective. In the treatment of spinocerebellar degeneration (SCD) SCA type 1 to SCA type 34, drug delivery to brainstem and/or cerebellum may be effective. In the treatment of dentatorubral-pallidoluysian atrophy (DRPLA), drug delivery to basal ganglion, brainstem and/or cerebellum may be effective. In the treatment of spinal and bulbar muscular atrophy (SBMA), drug delivery to brainstem and/or spinal cord may be effective. In the treatment of Friedreich's ataxia (FA), drug delivery to brainstem and/or cerebellum may be effective. In the treatment of Huntington's disease, drug delivery to striatum, frontal lobe, parietal lobe and/or basal ganglion may be effective. In the treatment of prion diseases (including mad cow disease and GSS), drug delivery to cerebral cortex, cerebral white matter, basal ganglion and/or substantia nigra may be effective. In the treatment of white matter encephalopathy, drug delivery to cerebral white matter may be effective. In the treatment of encephalitis (including viral, bacterial, fungal, and tuberculous encephalitis), and meningitis (including viral, bacterial, fungal, and tuberculous meningitis), drug delivery to the entire brain may be effective. In the treatment of metabolic encephalopathy, toxic encephalopathy, and trophopathic encephalopathy, drug delivery to the entire brain may be effective. In the treatment of white matter encephalopathy, drug delivery to cerebral white matter may be effective. In the treatment of cerebral infarction, cerebral hemorrhage, subarachnoid hemorrhage, moyamoya disease, and cerebral anoxia, drug delivery to the entire brain may be effective. In the treatment of white matter encephalopathy, drug delivery to cerebral white matter may be effective. In the treatment of diffuse axonal injury, drug delivery to cerebral white matter may be effective. In the treatment of head trauma, drug delivery to the entire brain may be effective. In the treatment of multiple sclerosis (MS), and neuromyelitis optica (NMO), drug delivery to cerebral white matter, cerebral cortex, optic nerve and/or spinal cord may be effective. In the treatment of myotonic dystrophy (DM1, DM2), drug delivery to skeletal muscle, cardiac muscle, cerebral cortex, and/or cerebral white matter may be effective. In the treatment of hereditary spastic paraparesis (HSP), drug delivery to parietal lobe and/or spinal cord may be effective. In the treatment of Fukuyama type muscular dystrophy, drug delivery to skeletal muscle, cerebral cortex and/or cerebral white matter may be effective. In the treatment of dementia with Lewy bodies (DLB), drug delivery to substantia nigra, striatum, occipital lobe, frontal lobe and/or parietal lobe may be effective. In the treatment of multiple system atrophy (MSA), drug delivery to striatum, basal ganglion, cerebellum, substantia nigra, frontal lobe and/or temporal lobe may be effective. In the treatment of Alexander's disease, drug delivery to cerebral white matter may be effective. In the treatment of CADASIL or CARASIL, drug delivery to cerebral white matter may be effective.

When a composition is applied by administration or ingestion, the administration amount or the ingestion amount may be appropriately selected according to the age (such as age in months and age in weeks), body weight, symptoms and health conditions of a subject, the type of the composition (such as medicine, food and beverage), or the like. The effective ingestion amount of the composition of the present invention for a subject in terms of the content of the nucleic acid complex may be from 0.00001 mg/kg/day to 10000 mg/kg/day, or from 0.001 mg/kg/day to 100 mg/kg/day. The composition may be applied by a single administration, or multiple administrations. In the case of multiple administrations, it may be administered daily or at appropriate time intervals (e.g., at intervals of 1 day, 2 days, 3 days, 1 week, 2 weeks, or 1 month), for example, for 2 to 20 times. A single dosage of the nucleic acid complex described above may be, for example, 0.001 mg/kg or more, 0.005 mg/kg or more, 0.01 mg/kg or more, 0.025 mg/kg or more, 0.1 mg/kg or more, 0.5 mg/kg or more, 1 mg/kg or more, 2.5 mg/kg or more, 0.5 mg/kg or more, 1.0 mg/kg or more, 2.0 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any amount within a range from 0.001 mg/kg to 500 mg/kg (e.g., 0.001 mg/kg, 0.01 mg/kg, 0.1 mg/kg, 1 mg/kg, 5 mg/kg, 10 mg/kg, 50 mg/kg, 100 mg/kg, or 200 mg/kg) may be selected as appropriate.

The nucleic acid complex of the present invention may be administered four times at a frequency of twice a week at a dose of from 0.01 to 10 mg/kg (e.g., about 6.25 mg/kg). Further, the nucleic acid complex may be administered two to four times at a frequency of once to twice a week, for example, administered twice at a frequency of twice a week, at a dose of from 0.05 to 30 mg/kg (e.g., about 25 mg/kg). By adopting such a dosing regimen (divided administration), the toxicity can be lowered and the load on a subject can be reduced compared to a single administration of a higher dose.

Although there is a limit (upper limit) on the amount crossing the BBB, the amount crossing the BCSFB, the amount crossing the CSFBB, the amount crossing the blood-nerve barrier (BNB), or the like by a single administration of a nucleic acid complex, it is considered that the inhibitory effect is produced additively in cells in the case of repeated administrations. That is, at a higher dose beyond the BBB crossing limit, the limit of the amount crossing BCSFB, the limit of the amount crossing CSFBB, the BNB crossing limit, or the like (e.g., 25 mg/kg or more), enhancement of the effectiveness is reduced when the amount of a single administration is increased, however the effectiveness is considered to be enhanced by performing repeated administrations at an administration interval of certain extent (e.g., more than half a day).

In a certain embodiment, the nucleic acid complex of the present invention has excellent properties as a medicine, e.g., is excellent in solubility in water, the second fluid for elution test of Japanese Pharmacopoeia, or the second fluid for disintegration test of Japanese Pharmacopoeia, is excellent in biokinetics (e.g., drug half-life in blood, intracerebral transferability, metabolic stability, and CYP inhibition), is low in toxicity (e.g., is more excellent as a medicine in terms of, e.g., acute toxicity, chronic toxicity, genetic toxicity, reproductive toxicity, cardiac toxicity, drug interaction, carcinogenicity, and phototoxicity), and also has few side effects (e.g., suppression of sedation and avoidance of laminar necrosis).

### (Drug delivery)

The composition of the present invention can deliver a specific drug to the nervous system, preferably to the central nervous system, further preferably to the brain (brain parenchyma) by binding the drug to the first nucleic acid strand and/or the second nucleic acid strand, utilizing the advantage that a nucleic acid complex of the first aspect or a single-stranded nucleic acid strand, which is contained as an active ingredient, can cross BBB and so on to be delivered efficiently to the central nervous system. There is no particular restriction on the drug to be delivered to the nervous system, and examples thereof include a peptide, a protein, and a nucleic acid drug as well as other organic compounds, such as an antitumor drug, a hormonal drug, an antibiotic, an antiviral drug, and an anti-inflammatory drug. The drug is preferably a small molecule drug. The meaning of a "small molecule drug" is well understood in the art. A small molecule drug typically refers to a drug having a molecular weight of less than 1,000 Da. The drug may be also a lipophilic drug. Examples of a nucleic acid drug includes, but not particularly limited to, ASO, antagomiR, splice-switching oligonucleotide, aptamer, single-stranded siRNA, microRNA, and pre-microRNA. The binding position and the type of binding of a drug in the second nucleic acid strand are as described above in connection with the binding of a C₂₂₋₃₅ alkyl group optionally substituted with a hydroxy group or an analog thereof to the second nucleic acid strand.

The composition of the present invention can be highly efficiently delivered to the central nervous system (preferably the brain (brain parenchyma)) to effectively modify or inhibit the expression of a target gene, or the level of a target transcriptional product, as disclosed in the following Examples. Consequently, provided is a method of decreasing the expression amount of a target transcriptional product in the central nervous system of a subject, comprising administering a composition comprising the above nucleic acid complex to a subject. This method may be a method for treating a central nervous system disease of a subject. Further provided is a method of delivering a drug to the central nervous system of a subject, comprising administering a composition comprising the above nucleic acid complex to a subject.

### Examples

The invention is further described in detail by way of the following Reference Examples and Examples, provided that these Examples do not restrict the present invention, and may be modified without departing from the scope of the present invention.

In the following Examples, "room temperature" usually indicates about 10°C to about 35°C. The ratio indicated with respect to a mixed solvent is a volume ratio unless otherwise specified. A percentage (%) indicates % by weight unless otherwise specified.

For the analysis of ¹H NMR, an ACD/SpecManager (product name) software, or the like was used. A proton peak of a hydroxy group, an amino group, or the like is sometimes not described when the peak is very shallow.

The meanings of the abbreviations used in Examples are as follow.
- M:: Molar concentration
- N:: Normality
- CDCl₃:: Deuterated chloroform
- ¹H NMR:: Proton nuclear magnetic resonance
- DIPEA:: N,N-diisopropylethylamine
- DMSO:: Dimethyl sulfoxide
- HATU:: O-(7-Azabenzotriazol-1-yl)-N,N,N' ,N' -tetramethyluronium hexafluorophosphate
- NMP: N-methyl-2-pyrrolidone
- PBS:: Phosphate-buffered saline
- TEAA:: Triethylamine acetate
- THF:: Tetrahydrofuran
- ODS:: Octadecyl-bound silica gel

The structures of oligonucleotides used in the following Examples are summarized in Table 1. Among the oligonucleotides used in Examples, ASO (Malat1) and Y13-cRNA (Malat1) were synthesized by GeneDesign Inc. (Osaka, Japan).

**[Table 1]**

| Double strand No. | Double-stranded nucleic acid agent name | Oligonucleotide name | 5'-Modification | Sequence (from 5' to 3') | SEQ ID NO: |
|---|---|---|---|---|---|
| n/a | n/a | ASO (Malat1) | | C*T*A*g*t*t*c*a*c*t*g*a*a*T*G*C | 1 |
| n/a | n/a | Y13-cRNA (Malat1) | Y13- | g*c*a*UUCAGUGAAC*u*a*g | 2 |
| 1 | Y1-HDO | ASO (Malat1) | | C*T*A*g*t*t*c*a*c*t*g*a*a*T*G*C | 1 |
| | | Y1-cRNA (Malat1) | Y1- | g*c*a*UUCAGUGAAC*u*a*g | 2 |
| 2 | Y2-HDO | ASO (Malat1) | | C*T*A*g*t*t*c*a*c*t*g*a*a*T*G*C | 1 |
| | | Y1-cRNA (Malat1) | Y2- | g*c*a*UUCAGUGAAC*u*a*g* | 2 |
| 3 | Y3-HDO | ASO (Malat1) | | C*T*A*g*t*t*c*a*c*t*g*a*a*T*G*C | 1 |
| | | Y3-cRNA (Malat1) | Y3- | g*c*a*UUCAGUGAAC*u*a*g* | 2 |
| 4 | Y4-HDO | ASO (Malat1) | | C*T*A*g*t*t*c*a*c*t*g*a*a*T*G*C | 1 |
| | | Y4-cRNA (Malat1) | Y4- | g*c*a*UUCAGUGAAC*u*a*g | 2 |
| 5 | Y5-HDO | ASO (Malat1) | | C*T*A*g*t*t*c*a*c*t*g*a*a*T*G*C | 1 |
| | | Y5-cRNA (Malat1) | Y5- | g*c*a*UUCAGUGAAC*u*a*g* | 2 |
| 6 | Y6-HDO | ASO (Malat1) | | C*T*A*g*t*t*c*a*c*t*g*a*a*T*G*C | 1 |
| | | Y6-cRNA (Malat1) | Y6- | g*c*a*UUCAGUGAAC*u*a*g | 2 |
| 7 | Y7-HDO | ASO (Malat1) | | C*T*A*g*t*t*c*a*c*t*g*a*a*T*G*C | 1 |
| | | Y7-cRNA (Malat1) | Y7- | g*c*a*UUCAGUGAAC*u*a*g* | 2 |
| 8 | Y8-HDO | ASO (Malat1) | | C*T*A*g*t*t*c*a*c*t*g*a*a*T*G*C | 1 |
| | | Y8-cRNA (Malat1) | Y8- | g*c*a*UUCAGUGAAC*u*a*g* | 2 |
| 9 | Y9-HDO | ASO (Malat1) | | C*T*A*g*t*t*c*a*c*t*g*a*a*T*G*C | 1 |
| | | Y9-cRNA (Malat1) | Y9- | g*c*a*UUCAGUGAAC*u*a*g | 2 |
| 10 | Y10-HDO | ASO (Malat1) | | C*T*A*g*t*t*c*a*c*t*g*a*a*T*G*C | 1 |
| | | Y10-cRNA(Malatl) | Y10- | g*c*a*UUCAGUGAAC*u*a*g* | 2 |
| 11 | Y11-HDO | ASO (Malat1) | | C*T*A*g*t*t*c*a*c*t*g*a*a*T*G*C | 1 |
| | | Y11-cRNA (Malat1) | Y11- | g*c*a*UUCAGUGAAC*u*a*g* | 2 |
| 12 | Y12-HDO | ASO (Malat1) | | C*T*A*g*t*t*c*a*c*t*g*a*a*T*G*C | 1 |
| | | Y12-cRNA (Malat1) | Y12- | g*c*a*UUCAGUGAAC*u*a*g | 2 |

| | | | | | |
|---|---|---|---|---|---|
| Upper case/underlined: LNA (C=5-methyl cytosine LNA), Lower case: DNA, Upper case: RNA, Lower case/underlined: 2'-OMe RNA, *: Phosphorothioate bond | | | | | |

The 5' end structure of the oligonucleotide Y13-cRNA (Malat1) shown in Table 1 is shown below. In the following Formula, the indication "oligo" represents an oligonucleotide.

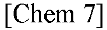

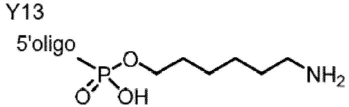

The 5' end structure of the oligonucleotide Y1-cRNA (Malat1) shown in Table 1 is shown below. In the following Formula, the indication "oligo" represents an oligonucleotide.

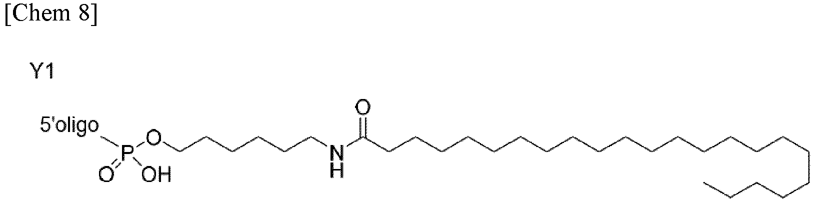

The 5' end structure of the oligonucleotide Y2-cRNA (Malat1) shown in Table 1 is shown below. In the following Formula, the indication "oligo" represents an oligonucleotide.

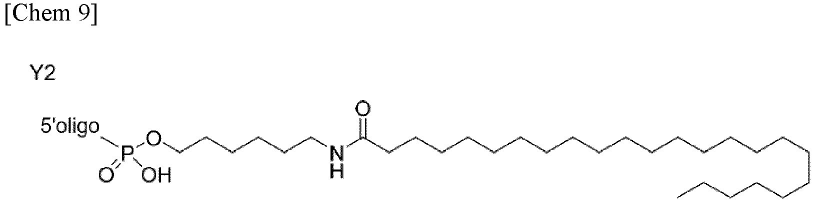

The 5' end structure of the oligonucleotide Y3-cRNA (Malat1) shown in Table 1 is shown below. In the following Formula, the indication "oligo" represents an oligonucleotide.

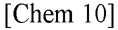

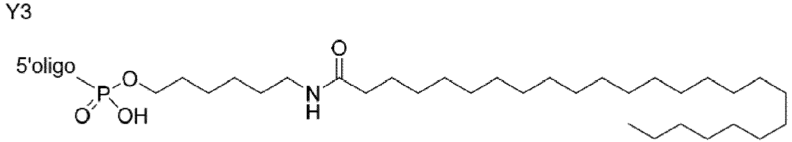

The 5' end structure of the oligonucleotide Y4-cRNA (Malat1) shown in Table 1 is shown below. In the following Formula, the indication "oligo" represents an oligonucleotide.

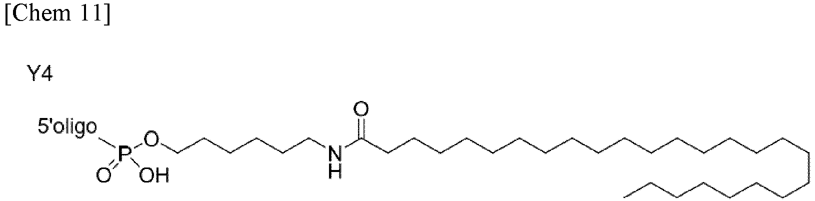

The 5' end structure of the oligonucleotide Y5-cRNA (Malat1) shown in Table 1 is shown below. In the following Formula, the indication "oligo" represents an oligonucleotide.

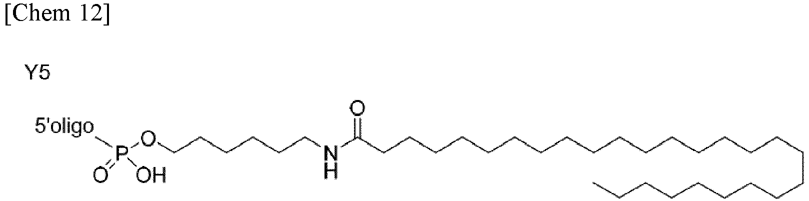

The 5' end structure of the oligonucleotide Y6-cRNA (Malat1) shown in Table 1 is shown below. In the following Formula, the indication "oligo" represents an oligonucleotide.

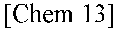

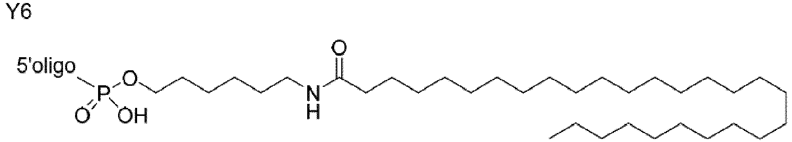

The 5' end structure of the oligonucleotide Y7-cRNA (Malat1) shown in Table 1 is shown below. In the following Formula, the indication "oligo" represents an oligonucleotide.

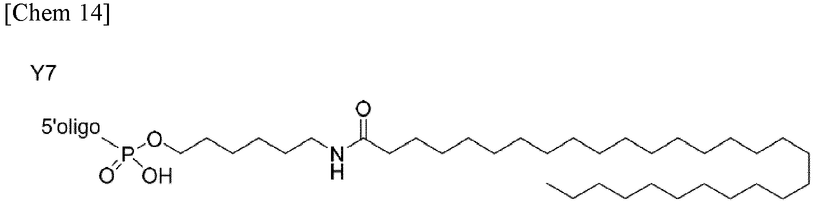

The 5' end structure of the oligonucleotide Y8-cRNA (Malat1) shown in Table 1 is shown below. In the following Formula, the indication "oligo" represents an oligonucleotide.

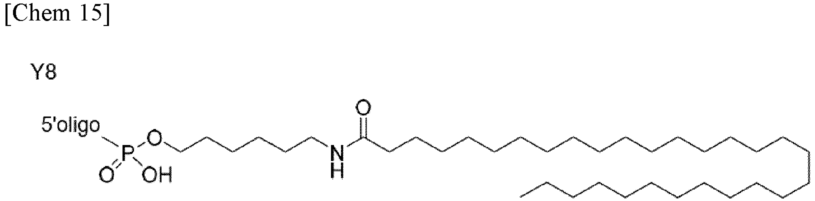

The 5' end structure of the oligonucleotide Y9-cRNA (Malat1) shown in Table 1 is shown below. In the following Formula, the indication "oligo" represents an oligonucleotide.

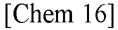

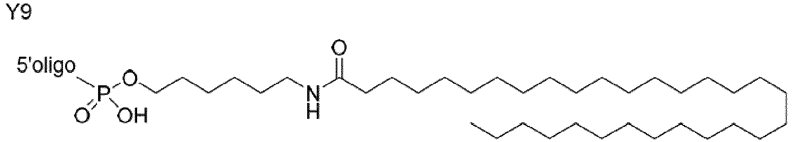

The 5' end structure of the oligonucleotide Y10-cRNA (Malat1) shown in Table 1 is shown below. In the following Formula, the indication "oligo" represents an oligonucleotide.

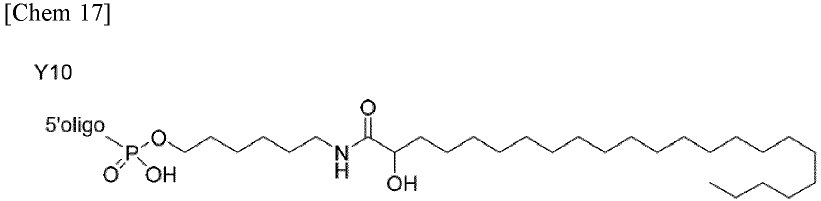

The 5' end structure of the oligonucleotide Y11-cRNA (Malat1) shown in Table 1 is shown below. In the following Formula, the indication "oligo" represents an oligonucleotide.

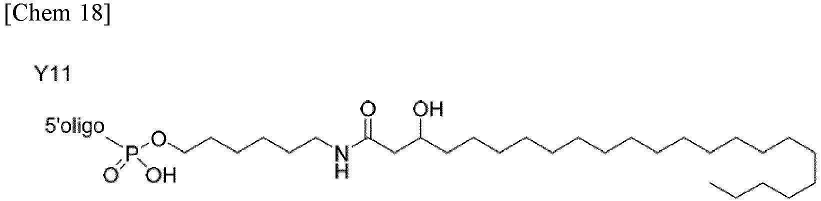

The 5' end structure of the oligonucleotide Y12-cRNA (Malat1) shown in Table 1 is shown below. In the following Formula, the indication "oligo" represents an oligonucleotide.

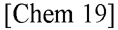

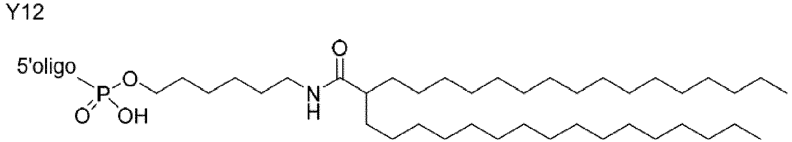

Other compounds used in Examples are listed in the following Table. Commercial products may be used as they are, respectively, or they may be produced by a publicly known method or a method conforming thereto.

**[Table 2]**

| Compound name | Structural formula |
|---|---|
| IY1 | 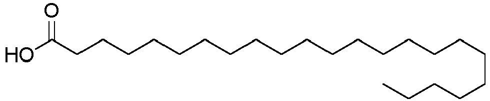 |
| IY2 | 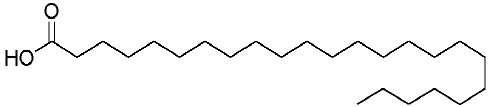 |
| IY3 | 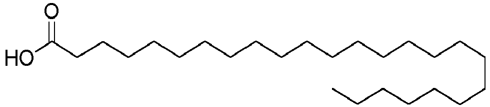 |
| IY4 | 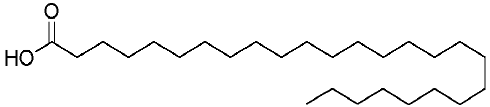 |
| IY5 | 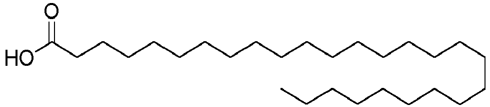 |
| IY6 | 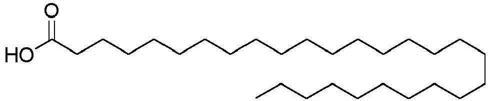 |
| IY7 | 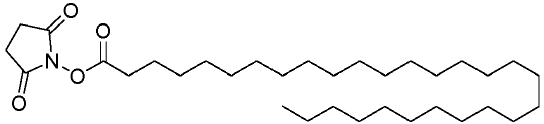 |
| IY8 | 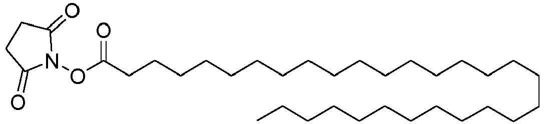 |
| IY9 | 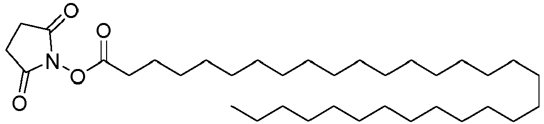 |
| IY10 | 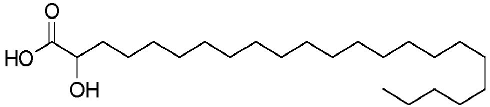 |
| IY11 | 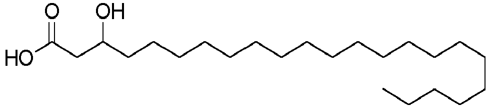 |

### Example 1

### A) Synthesis of cRNA bound to Y1- at 5' end (Y1-cRNA (Malat1))

A 50 mM NMP solution of IY1 (1200 µL), a 75 mM NMP solution of HATU (1200 µL), and a 150 mM NMP solution of DIPEA (1200 µL) were mixed in a microtube, stirred, precipitated, and then allowed to react at room temperature for 15 min. Thereto an aqueous solution of the RNA strand (Y13-cRNA (Malat1)) listed in Table 1 (3000 nmol), NMP (3150 µL), distilled water (153 µL), and DIPEA (94 µL) were added. Then the mixture was stirred, precipitated, and then allowed to react at room temperature for 1 hour. The reaction solution was purified in an ODS column (column: Purif-Pack^{®}-EX ODS-50 size 60, produced by Shoko Science Co., Ltd., mobile phase: TEAA/acetonitrile), and desalinated by ultrafiltration (Amicon Ultra 3 kDa, produced by Merck Millipore, distilled water). To the obtained solution, ten fold volume of 1 M meglumine acetate was added, and the mixture was stirred thoroughly and left standing for 5 min for ion exchange. Thereafter, it was desalinated by ultrafiltration (Amicon Ultra 3 kDa, produced by Merck Millipore, distilled water). The final product was filtrated through a 0.20 µm membrane filter and freeze-dried to yield 1446 nmol of the captioned compound.

### B) Synthesis of double-stranded nucleic acid agent Y1-HDO

A 16mer single-stranded LNA/DNA gapmer (ASO (Malat1)) targeting Malat1 non-coding RNA comprises three LNA nucleoside at the 5' end and three LNA nucleoside at the 3' end, and ten DNA nucleosides between them. This LNA/DNA gapmer has a base sequence complementary to 1316-1331 of the murine Malat1 non-coding RNA (GenBank Accession number NR 002847, SEQ ID NO: 3). The ASO (Malat1) (first nucleic acid strand) and the Y1-cRNA (Malat1) (second nucleic acid strand) obtained in the previous step A were mixed at equiomolar amount, and the mixed liquid heated at 70°C for 5 min. Then, the mixed liquid was slowly cooled to room temperature, to yield the Y1-conjugated heteroduplex oligonucleotide (Y1-HDO), which was a double-stranded nucleic acid agent as described above.

### Example 2

### A) Synthesis of cRNA bound to Y2- at 5' end (Y2-cRNA (Malat1))

The captioned compound in an amount of 1245 nmol was produced in the same manner as in step A of Example 1 using an aqueous solution of the RNA strand (Y13-cRNA (Malat1)) (3000 nmol) as shown in Table 1, and IY2.

### B) Synthesis of double-stranded nucleic acid agent Y2-HDO

The Y2-conjugated heteroduplex oligonucleotide (Y2-HDO), which was a double-stranded nucleic acid agent, was prepared in the same manner as in step B of Example 1, using the ASO (Malat1) (first nucleic acid strand) and the Y2-cRNA (Malat1) (second nucleic acid strand) obtained in the previous step A.

### Example 3

### A) Synthesis of cRNA bound to Y3- at 5' end (Y3-cRNA (Malat1))

The captioned compound in an amount of 702 nmol was produced in the same manner as in step A of Example 1 using an aqueous solution of the RNA strand (Y13-cRNA (Malat1)) (3000 nmol) as shown in Table 1, and IY3.

### B) Synthesis of double-stranded nucleic acid agent Y3-HDO

The Y3-conjugated heteroduplex oligonucleotide (Y3-HDO), which was a double-stranded nucleic acid agent, was prepared in the same manner as in step B of Example 1, using the ASO (Malat1) (first nucleic acid strand) and Y3-cRNA (Malat1) (second nucleic acid strand) obtained in the previous step A.

### Example 4

### A) Synthesis of cRNA bound to Y4- at 5' end (Y4-cRNA (Malat1))

The captioned compound in an amount of 1277 nmol was produced in the same manner as in step A of Example 1 using an aqueous solution of the RNA strand (Y13-cRNA (Malat1)) (3000 nmol) as shown in Table 1, and IY4.

### B) Synthesis of double-stranded nucleic acid agent Y4-HDO

The Y4-conjugated heteroduplex oligonucleotide (Y4-HDO), which was a double-stranded nucleic acid agent, was prepared in the same manner as in step B of Example 1, using the ASO (Malat1) (first nucleic acid strand) and Y4-cRNA (Malat1) (second nucleic acid strand) obtained in the previous step A.

### Example 5

### A) Synthesis of cRNA bound to Y5- at 5' end (Y5-cRNA (Malat1))

The captioned compound in an amount of 1330 nmol was produced in the same manner as in step A of Example 1 using an aqueous solution of the RNA strand (Y13-cRNA (Malat1)) (3000 nmol) as shown in Table 1, and IY5.

### B) Synthesis of double-stranded nucleic acid agent Y5-HDO

The Y5-conjugated heteroduplex oligonucleotide (Y5-HDO), which was a double-stranded nucleic acid agent, was prepared in the same manner as in step B of Example 1, using the ASO (Malat1) (first nucleic acid strand) and Y5-cRNA (Malat1) (second nucleic acid strand) obtained in the previous step A.

### Example 6

### A) Synthesis of cRNA bound to Y6- at 5' end (Y6-cRNA (Malat1))

The captioned compound in an amount of 713 nmol was produced in the same manner as in step A of Example 1 using an aqueous solution of the RNA strand (Y13-cRNA (Malat1)) (3000 nmol) as shown in Table 1, and IY6. Here, the solvent used for dissolving the IY6 was

### THF.

### B) Synthesis of double-stranded nucleic acid agent Y6-HDO

The Y6-conjugated heteroduplex oligonucleotide (Y6-HDO), which was a double-stranded nucleic acid agent, was prepared in the same manner as in step B of Example 1, using the ASO (Malat1) (first nucleic acid strand) and Y6-cRNA (Malat1) (second nucleic acid strand) obtained in the previous step A.

### Example 7

### A) Preparation of NHS-modified fatty acid IY7

A mixture of nonacosylic acid (256 mg) and a thionyl chloride solution (1.49 mL) was stirred with heating at reflex for 30 min. After the reaction solution was cooled to room temperature, the solvent was distilled off under reduced pressure, and the residue was added to water and azeotropically boiled with toluene twice. After the resulting residue was dissolved in pyridine (2.0 mL), 1-hydroxypyrrolidine-2,5-dione (77 mg) was added thereto, and the mixture was stirred with heating at reflex for 30 min. After the reaction solution was cooled to room temperature, 1 N hydrochloric acid, THF, and ethyl acetate were added, and then the resulting solid was collected by filtration, and 232 mg of the captioned compound was obtained. The NMR results of the obtained compound are as follows.

¹H NMR (300 MHz, CDCl3) δ 0.88 (3H, brs), 1.11-1.85 (52H, m), 2.54-2.67 (2H, m), 2.84 (4H, brs).

### B) Synthesis of cRNA bound to Y7- at 5' end (Y7-cRNA (Malat1))

In an aqueous solution of the RNA strand (Y13-cRNA (Malat1)) listed in Table 1 (672 µL, 4000 nmol), a 50 mM NMP solution of IY7 (800 µL), distilled water (328 µL), NMP (7200 µL), ×10 PBS (1000 µL), and DIPEA (125 µL) were mixed in the mentioned order in a microtube, stirred, precipitated, and then allowed to react at 70°C for one and a half hours. The reaction solution was purified in an ODS column (column: Purif-Pack^{®}-EX ODS-50 size 60, produced by Shoko Science Co., Ltd., mobile phase: TEAA/acetonitrile), and desalinated by ultrafiltration (Amicon Ultra 3 kDa, produced by Merck Millipore, distilled water). To the obtained solution, ten times the amount of 1 M meglumine acetate was added, and the mixture was stirred thoroughly and left standing for 5 min for ion exchange. Thereafter, it was desalinated by ultrafiltration (Amicon Ultra, 3 kDa, produced by Merck Millipore, distilled water). The final product was filtrated through a 0.20 µm membrane filter and freeze-dried to yield 1692 nmol of the captioned compound.

### C) Synthesis of double-stranded nucleic acid agent Y7-HDO

The Y7-conjugated heteroduplex oligonucleotide (Y7-HDO), which was a double-stranded nucleic acid agent, was prepared in the same manner as in step B of Example 1, using the ASO (Malat1) (first nucleic acid strand) and the Y7-cRNA (Malat1) (second nucleic acid strand) obtained in the previous step B.

### Example 8

### A) Preparation of NHS-modified fatty acid IY8

The captioned compound in an amount of 177 mg was obtained in the same manner as in step A of Example 7 using melissic acid (188 mg). The NMR results of the obtained compound are as follows.

1H NMR (300 MHz, CDCl3) δ 0.88 (3H, t, J = 6.4 Hz), 1.11-1.87 (54H, m), 2.60 (2H, t, J = 7.2 Hz), 2.84 (4H, s).

### B) Synthesis of cRNA bound to Y8- at 5' end (Y8-cRNA (Malat1))

The captioned compound in an amount of 1089 nmol was obtained in the same manner as in step B of Example 7 using an aqueous solution of the RNA strand (Y13-cRNA (Malat1)) shown in Table 1 (4000 nmol), and IY8.

### C) Synthesis of double-stranded nucleic acid agent Y8-HDO

The Y8-conjugated heteroduplex oligonucleotide (Y8-HDO), which was a double-stranded nucleic acid agent, was prepared in the same manner as in step B of Example 1, using the ASO (Malat1) (first nucleic acid strand) and the Y8-cRNA (Malat1) (second nucleic acid strand) obtained in the previous step B.

### Example 9

### A) Preparation of NHS-modified fatty acid IY9

The captioned compound in an amount of 66 mg was obtained in the same manner as in step A of Example 7 using hentriacontanoic acid (100 mg). The NMR results of the obtained compound are as follows.

1H NMR (300 MHz, CDCl3) δ 0.83-0.94 (3H, m), 1.20-1.80 (56H, m), 2.60 (2H, t, J = 7.5 Hz), 2.83 (4H, s).

### B) Synthesis of cRNA bound to Y9- at 5' end (Y9-cRNA (Malat1))

The captioned compound in an amount of 955 nmol was obtained in the same manner as in step B of Example 7 using an aqueous solution of the RNA strand (Y13-cRNA (Malat1)) shown in Table 1 (3500 nmol), and a THF solution of IY9.

### C) Synthesis of double-stranded nucleic acid agent Y9-HDO

The Y9-conjugated heteroduplex oligonucleotide (Y9-HDO), which was a double-stranded nucleic acid agent, was prepared in the same manner as in step B of Example 1, using the ASO (Malat1) (first nucleic acid strand) and the Y9-cRNA (Malat1) (second nucleic acid strand) obtained in the previous step B.

### Example 10

### A) Synthesis of cRNA bound to Y10- at 5' end (Y10-cRNA (Malat1))

The captioned compound in an amount of 2159 nmol was produced in the same manner as in step A of Example 1 using an aqueous solution of the RNA strand (Y13-cRNA (Malat1)) (3000 nmol) as shown in Table 1, and IY10. Here, a mixture of THF, DMSO and water was used as the solvent.

### B) Synthesis of double-stranded nucleic acid agent Y10-HDO

The Y10-conjugated heteroduplex oligonucleotide (Y10-HDO), which was a double-stranded nucleic acid agent, was prepared in the same manner as in step B of Example 1, using the ASO (Malat1) (first nucleic acid strand) and Y10-cRNA (Malat1) (second nucleic acid strand) obtained in the previous step A.

### Example 11

### A) Synthesis of cRNA bound to Y11- at 5' end (Y11-cRNA (Malat1))

The captioned compound in an amount of 549 nmol was produced in the same manner as in step A of Example 1 using an aqueous solution of the RNA strand (Y13-cRNA (Malat1)) (3000 nmol) as shown in Table 1, and IY11. Here, a mixture of THF, DMSO and water was used as the solvent.

### B) Synthesis of double-stranded nucleic acid agent Y11-HDO

The Y11-conjugated heteroduplex oligonucleotide (Y11-HDO), which was a double-stranded nucleic acid agent, was prepared in the same manner as in step B of Example 1, using the ASO (Malat1) (first nucleic acid strand) and Y11-cRNA (Malat1) (second nucleic acid strand) obtained in the previous step A.

### Example 12

### A) Synthesis of cRNA bound to Y12- at 5' end (Y12-cRNA (Malat1))

The captioned compound in an amount of 977 nmol was produced in the same manner as in step A of Example 1 using an aqueous solution of the RNA strand (Y13-cRNA (Malat1)) (3000 nmol) as shown in Table 1, and a THF solution of IY12.

### B) Synthesis of double-stranded nucleic acid agent Y12-HDO

The Y12-conjugated heteroduplex oligonucleotide (Y12-HDO), which was a double-stranded nucleic acid agent, was prepared in the same manner as in step B of Example 1, using the ASO (Malat1) (first nucleic acid strand) and Y12-cRNA (Malat1) (second nucleic acid strand) obtained in the previous step A.

### Example 13

### (A) In vivo experiments

Male 7-week-old C57BL/6J mice (Charles River Laboratories Japan Inc.) were used as experimental animals, and two to four mice per group were subjected to experiments. In the experimental group, a solution containing a nucleic acid was administered in a single dose of 5 mL/kg intravenously to the tail vein of a mouse. In the control group, the solvent used for preparing the nucleic acid solution (5% glucose solution) was intravenously administered to the mice in the same procedure as in the experimental group.

### (B) Expression analysis

Seventy-two hours after the administration of the nucleic acid solution, each mouse was anesthetized by intraperitoneal administration of 50 mg/kg of pentobarbital, and sacrificed after collection of the blood sample. The brain (cerebral cortex) was isolated. For extraction of total RNA from the brain tissues, an RNA extraction reagent ISOGEN (Nippon Gene Co., Ltd.) was used. The isolated brain tissue was homogenyzed in the ISOGEN solution, and then separated RNA fraction with chloroform. Thereafter, a nucleic acid separation system QuickGene RNA tissue kit SII (Kurabo Industries Ltd.) was used. For cDNA synthesis from the total RNA, a ReverTra Ace qPCR RT Kit (Toyobo Co., Ltd.) was used, and quantitative PCR was performed using a THUNDERBIRD qPCR Mix (Toyobo Co., Ltd.). For the quantitative PCR, a fluorescent probe method was applied, and as fluorescent probes, murine Malat1 (Integrated DNA Technologies), and murine Gapdh (Thermo Fisher Scientific) were used. Gene fragment amplification reaction conditions for the quantitative PCR were based on the protocol of the aforedescribed THUNDERBIRD qPCR Mix (Toyobo Co., Ltd.). The expression amounts of murine Malat1 and Gapdh (internal standard gene) were calculated using relative calibration curves and the relative expression level was calculated as Malat1/Gapdh. The mean relative expression level was calculated from the results of two mice per group. With the mean relative expression level of the control group being 100%, the mean relative expression level of the experimental group was calculated as the relative Malat1 ncRNA expression level.

### (C) Results

The results of Example 13 are shown in Table 3. In the table, the dosage indicates the amount of the ASO (Malat1).

All of the double strand numbers 1 to 12 inhibited expression of Malat1 non-coding RNA in the cerebral cortex. These results demonstrated that the nucleic acid complex of the present invention can be delivered to the brain, where it can produce an antisense effect.

### [Table 3]

**Table 3: Effect of suppression of the expression of Malat1 ncRNA in cerebral cortex by double-stranded nucleic acid agent**

| Double strand No. | Dosage | Relative Malat1 ncRNA expression level |
|---|---|---|
| 1 | 50 mg/kg | 49% |
| 2 | 50 mg/kg | 52% |
| 3 | 50 mg/kg | 51% |
| 4 | 50 mg/kg | 66% |
| 5 | 50 mg/kg | 48% |
| 6 | 50 mg/kg | 67% |
| 7 | 50 mg/kg | 69% |
| 8 | 50 mg/kg | 87% |
| 9 | 50 mg/kg | 65% |
| 10 | 10 mg/kg | 71% |
| 10 | 20 mg/kg | 51% |
| 11 | 10 mg/kg | 68% |
| 12 | 50 mg/kg | 71% |

All publications, patents, and patent applications cited herein are incorporated herein directly by reference.

## Claims

1. A nucleic acid complex comprising a first nucleic acid strand and a second nucleic acid strand or a salt thereof, wherein:
the first nucleic acid strand comprises a base sequence capable of hybridizing to at least a part of a target transcriptional product, and has an antisense effect on the target transcriptional product;
the second nucleic acid strand comprises a base sequence complementary to the first nucleic acid strand, and is bound to a C₂₂₋₃₅ alkyl group optionally substituted with a hydroxy group, or an analog thereof; and
the first nucleic acid strand is annealed to the second nucleic acid strand.

2. The nucleic acid complex or a salt thereof according to claim 1, wherein said C₂₂₋₃₅ alkyl group optionally substituted with a hydroxy group or the analog thereof is an unsubstituted linear C₂₂₋₂₄ or linear C₂₆ alkyl group.

3. The nucleic acid complex or a salt thereof according to claim 1 or 2, wherein the second nucleic acid strand is bound to the C₂₂₋₃₅ alkyl group optionally substituted with a hydroxy group or an analog thereof via a linker represented by Formula I.

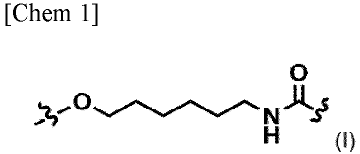

4. The nucleic acid complex or a salt thereof according to any one of claims 1 to 3, wherein said first nucleic acid strand comprises at least four contiguous deoxyribonucleosides.

5. The nucleic acid complex or a salt thereof according to any one of claims 1 to 4, wherein said first nucleic acid strand is a gapmer.

6. The nucleic acid complex or a salt thereof according to any one of claims 1 to 5, wherein said second nucleic acid strand comprises at least four contiguous ribonucleosides complementary to the at least four contiguous deoxyribonucleosides in said first nucleic acid strand.

7. The nucleic acid complex or a salt thereof according to any one of claims 1 to 4, wherein said first nucleic acid strand is a mixmer.

8. The nucleic acid complex or a salt thereof according to any one of claims 1 to 7, wherein said first nucleic acid strand is 13 to 20 bases in length.

9. The nucleic acid complex or a salt thereof according to any one of claims 1 to 8, wherein said second nucleic acid strand does not comprise a natural ribonucleoside.

10. The nucleic acid complex or a salt thereof according to any one of claims 1 to 9, wherein the nucleic acid portion of said second nucleic acid strand consists of deoxyribonucleosides and/or sugar-modified nucleosides linked by a modified or unmodified internucleoside bond.

11. A composition for regulating expression or editing of a target transcriptional product in the central nervous system, comprising the nucleic acid complex or a salt thereof according to any one of claims 1 to 10.

12. The composition according to claim 11, for treating a central nervous system disease.

13. A composition for delivering a drug to the central nervous system, comprising the nucleic acid complex or a salt thereof according to any one of claims 1 to 10.

14. The composition according to any one of claims 11 to 13, wherein said central nervous system is selected from the group consisting of cerebral cortex, basal ganglion, cerebral white matter, diencephalon, brainstem, cerebellum, and spinal cord.

15. The composition according to any one of claims 11 to 13, wherein said central nervous system is selected from the group consisting of frontal lobe, temporal lobe, hippocampus, parahippocampal gyrus, parietal lobe, occipital lobe, striatum, globus pallidus, claustrum, thalamus, subthalamic nucleus, midbrain, substantia nigra, pons, medulla oblongata, cerebellar cortex, cerebellar nucleus, cervical spinal cord, thoracic spinal cord, and lumbar spinal cord.

16. The composition according to any one of claims 11 to 15, for intravenous administration or subcutaneous administration.

17. The composition according to any one of claims 11 to 16, comprising 5 mg/kg or more of said nucleic acid complex or a salt thereof in a single dose.

18. The composition according to any one of claims 11 to 17, wherein said nucleic acid complex or a salt thereof transitions from blood to the brain.
